# EUROPÄISCHE PATENTSCHRIFT

(11) **EP 0 661 281 B1**
(45) Veröffentlichungstag und Bekanntmachung des Hinweises auf die Patenterteilung: **01.09.1999**
(21) Anmeldenummer: 94810734.7
(22) Anmeldetag: 19.12.1994
(51) Int. Cl.: C07D 317/22, C07D 317/30, C07D 407/04, C07D 405/04, A01N 43/28

(54) **Substituierte Phenylether als Pestizide**
Substituted phenylethers as pesticides
Phényléthers substitués comme pesticides

(30) Priorität: 30.12.1993 CH 390593
(43) Veröffentlichungstag der Anmeldung: 05.07.1995
(73) Patentinhaber: Novartis AG, 4058 Basel (CH)
(72) Erfinder: Karrer, Friedrich Dr., CH-4800 Zofingen (CH)

(56) Entgegenhaltungen:
- EP-A- 0 559 612
- US-A- 4 590 282

## Beschreibung

Die Erfindung betrifft Verbindungen der Formel 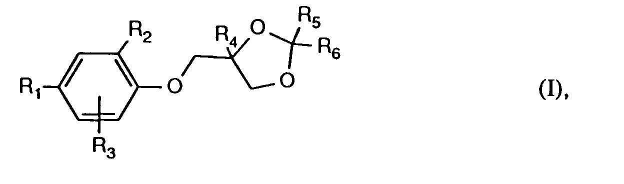 worin
R₁ gegebenenfalls substituiertes C₁-C₈-Alkyl, gegebenenfalls substituiertes C₃-C₈-Cycloalkyl, gegebenenfalls substituiertes C₂-C₈-Alkenyl, gegebenenfalls substituiertes C₂-C₈-Alkinyl, gegebenenfalls substituiertes C₁-C₈-Alkoxy, gegebenenfalls substituiertes C₃-C₈-Cycloalkoxy, gegebenenfalls substituiertes C₂-C₈-Alkenyloxy, gegebenenfalls substituiertes C₂-C₈-Alkinyloxy oder gegebenenfalls substituiertes C₁-C₈-Alkylthio;
R₂ Chlor oder Brom;
R₃ H, Halogen oder Methyl;
R₄ H oder Methyl;
R₅ C₁-C₈-Alkyl, C₃-C₆-Cycloalkyl, C₂-C₅-Alkenyl, C₂-C₅-Alkinyl, C₁-C₃-Alkoxy-C₁-C₃-alkyl, C₁-C₄-Cyanalkyl, C₁-C₆-Halogenalkyl, C₂-C₅-Halogenalkenyl, C₂-C₃-Halogenalkinyl, C₁-C₃-Alkoxycarbonyl, Trifluormethylphenyl, Benzyl oder substituiertes Benzyl, wobei die Substituenten ausgewählt sind aus der Gruppe, bestehend aus C₁-C₃-Alkyl, C₁-C₃-Alkoxy und Halogen; und
R₆ H, Pyridyl, Halogenpyridyl, Furyl, Thienyl oder R₅ und R₆ zusammen geradkettiges C₃-C₅-Alkylen bedeuten;
ein Verfahren zur Herstellung und die Verwendung dieser Verbindungen, Schädlingsbekämpfungsmittel, deren Wirkstoff aus diesen Verbindungen ausgewählt ist, ein Verfahren zur Herstellung und die Verwendung dieser Mittel und Zwischenprodukte für die Herstellung der Verbindungen der Formel I.

In der Literatur werden gewisse Dioxolanderivate als insektizid wirkende Wirkstoffe in Schädlingsbekämpfungsmitteln vorgeschlagen. So beschreibt US-A 4,590,282 ähnlich strukturierte Dioxolane sowie Benzodioxine zur Verwendung als Pestizide. Die biologischen Eigenschaften dieser bekannten Verbindungen vermögen auf den, Gebiet der Schädlingsbekämpfung jedoch nicht voll zu befriedigen, weshalb das Bedürfnis besteht, weitere Verbindungen mit schädlingsbekämpfenden Eigenschaften, insbesondere zur Bekämpfung von Insekten und Vertretern der Ordnung Acarina, zur Verfügung zu stellen, wobei diese Aufgabe erfindungsgemäss durch die Bereitstellung der vorliegenden Verbindungen I gelöst wird.

Die vor- und nachstehend verwendeten Allgemeinbegriffe haben, sofern nicht abweichend definiert, die nachfolgend aufgeführten Bedeutungen.

Kohlenstoffhaltige Gruppen und Verbindungen enthalten, sofern nicht abweichend definiert, jeweils 1 bis und mit 8, vorzugsweise 1 bis und mit 6, insbesondere 1 oder 2, Kohlenstoffatome.

Alkyl - als Gruppe per se sowie als Strukturelement von anderen Gruppen und Verbindungen, wie von Phenylalkyl, Alkylphenyl, Halogenalkyl, Alkoxy, Alkoxyalkyl, Alkoxyalkoxy, Halogenalkoxy, Alkylthio, Alkylthioalkyl, Alkylthioalkoxy und Alkylthioalkylthio, - ist, jeweils unter gebührender Berücksichtigung der von Fall zu Fall umfassten Anzahl der in der entsprechenden Gruppe oder Verbindung enthaltenen Kohlenstoffatome, entweder geradkettig, d. h. Methyl, Ethyl, Propyl, Butyl, Pentyl, Hexyl, Heptyl oder Octyl, oder verzweigt, z. B. Isopropyl, Isobutyl, sek.-Butyl, tert.-Butyl, Isopentyl, Neopentyl, Isohexyl, Isoheptyl oder Isooctyl. Beispiele für Alkoxyalkyl sind Methoxymethyl, Ethoxymethyl, Ethoxyethyl oder 2-Methoxybutyl; für Alkoxyalkoxy Methoxyethoxy oder Ethoxyethoxy; für Alkylthioalkyl Ethylthiomethyl oder Isobutylthiomethyl; für Alkylthioalkoxy Ethylthioethoxy oder i-Propylthioethoxy; für Alkylthioalkylthio Methylthioethylthio oder i-Propylthioethylthio.

Alkenyl, Alkenyloxy, Alkenyloxyalkyl, Halogenalkenyl, Halogenalkenyloxy, Halogenalkenyloxyalkyl, Alkinyl, Alkinyloxy, Alkinyloxyalkyl, Halogenalkinyl, Halogenalkinyloxy und Halogenalkinyloxyalkyl sind geradkettig oder verzweigt und enthalten jeweils zwei oder vorzugsweise eine ungesättigte Kohlenstoff-Kohlenstoff-Bindung(en). Beispiele für Alkenyloxyalkyl sind Allyloxymethyl oder 2-Buten-1-yloxymethyl; für Alkinyloxyalkyl 2-Propin-1-yloxymethyl oder 2-Pentin-1-yloxymethyl.

Cycloalkyl ist Cyclopropyl, Cyclobutyl, Cyclopentyl, Cyclohexyl, Cycloheptyl oder Cyclooctyl, vorzugsweise Cyclopropyl oder Cyclohexyl.

C₁-C₃-Alkylendioxy ist -O-CH₂-O-, -O-CH₂-CH₂-O- oder -O-CH₂-CH₂-CH₂-O-, vorzugsweise -O-CH₂-O-.

Geradkettiges C₃-C₅-Alkylen ist Trimethylen, Tetramethylen oder Pentamethylen.

C₁-C₄-Cyanalkyl ist Cyanmethyl, Cyanethyl, Cyanpropyl oder Cyanisopropyl, vor allem Cyanmethyl.

Halogen - als Gruppe per se sowie als Strukturelement von anderen Gruppen und Verbindungen, wie von Halogenalkyl und Halogenalkoxy, - ist Fluor, Chlor, Brom oder Iod, insbesondere Fluor, Chlor oder Brom, vor allem Chlor oder Brom.

Halogensubstituierte kohlenstoffhaltige Gruppen und Verbindungen, wie Halogenalkyl, Halogenalkylthio, Halogenalkoxy, Halogenalkenyl, Halogenalkenyloxy, Halogenalkenyloxyalkyl, Halogenalkinyl, Halogenalkinyloxy, Halogenalkinyloxyalkyl und Halogenallyloxy, können teilweise halogeniert oder perhalogeniert sein, wobei im Falle von Mehrfach-Halogenierung die Halogensubstituenten gleich oder verschieden sein können. Beispiele für Halogenalkyl - als Gruppe per se sowie als Strukturelement von anderen Gruppen und Verbindungen, wie von Halogenalkoxy, - sind das ein-bis dreifach durch Fluor, Chlor und/oder Brom substituierte Methyl, wie CHF₂ oder CF₃; das ein-bis fünffach durch Fluor, Chlor und/oder Brom substituierte Ethyl, wie CH₂CF₃, CF₂CF₃, CF₂CCl₃, CF₂CHCl₂, CF₂CHF₂, CF₂CFCl₂, CF₂CHBr₂, CF₂CHClF, CF₂CHBrF oder CClFCHClF; das ein- bis siebenfach durch Fluor, Chlor und/oder Brom substituierte Propyl oder Isopropyl, wie CH₂CHBrCH₂Br, CF₂CHFCF₃, CH₂CF₂CF₃, CF₂CF₂CF₃ oder CH(CF₃)₂; und das ein- bis neunfach durch Fluor, Chlor und/oder Brom substituierte Butyl oder eines seiner Isomeren, wie CF(CF₃)CHFCF₃, CF₂(CF₂)₂CF₃ oder CH₂(CF₂)₂CF₃. Beispiele für Halogenalkenyl sind 2,2-Difluorethenyl, 2,2-Dichlorethenyl, 3-Chlor-2-allyl, 3,3-Dichlor-2-allyl und 2,3-Dibrom-2-allyl. Beispiele für Halogenalkinyl sind 3-Chlor-2-propinyl, 1,3-Dichlor-2-propinyl und 1,3-Dibrom-2-propinyl.

Bevorzugte Ausführungsformen im Rahmen der Erfindung sind:
(1) Eine Verbindung der Formel I, worin
   - R₁: C₁-C₈-Alkyl, C₃-C₈-Cycloalkyl, C₂-C₈-Alkenyl, C₂-C₈-Alkinyl, C₁-C₈-Alkoxy, C₃-C₈-Cycloalkoxy, C₂-C₈-Alkoxy-C₁-C₄-alkyl, C₁-C₈-Alkoxy-C₂-C₄-alkoxy-C₁-C₄-alkyl, C₁-C₈-Alkoxy-C₂-C₄-alkoxy, C₄-C₈-Cycloalkoxy-C₁-C₃-alkyl, C₃-C₈-Alkenyloxy, C₃-C₈-Alkenyloxy-C₁-C₃-alkyl, C₃-C₈-Alkinyloxy, C₃-C₈-Alkinyloxy-C₁-C₃-alkyl, C₃-C₈-Halogenalkyl, C₃-C₈-Halogenalkenyl, C₃-C₈-Halogenalkinyl, C₃-C₈-Halogenalkoxy, C₃-C₈-Halogenalkoxy-C₁-C₃-alkyl, C₃-C₈-Halogenalkenyloxy, C₃-C₈-Halogenalkenyloxy-C₁-C₃-alkyl, C₃-C₈-Halogenalkinyloxy, C₃-C₈-Halogenalkinyloxy-C₁-C₃-alkyl, C₃-C₈-Alkylthio, C₃-C₈-Alkylthio-C₁-C₃- alkyl, C₂-C₈-Alkylthio-C₂-C₃-alkoxy, C₂-C₈-Alkylthio-C₂-C₃-alkoxy-C₁-C₄-alkyl, C₂-C₈-Alkylthio-C₂-C₃-alkylthio, C₂-C₈-Alkylthio-C₂-C₃-alkylthio-C₁-C₄-alkyl, C₂-C₈-Alkoxy-C₂-C₃-alkylthio oder C₂-C₈-Alkoxy-C₂-C₃-alkylthio-C₁-C₄-alkyl;
   vor allem C₃-C₆-Alkyl, C₃-C₆-Cycloalkyl, C₃-C₆-Alkenyl, C₃-C₆-Alkinyl, C₃-C₈-Alkoxy, C₃-C₆-Cycloalkoxy, C₂-C₆-Alkoxy-C₁-C₄-alkyl, C₁-C₆-Alkoxy-C₂-C₄-alkoxy, C₄-C₆-Cycloalkoxy-C₁-C₃-alkyl, C₃-C₆-Alkenyloxy, C₃-C₆-Alkenyloxy-C₁-C₃-alkyl, C₃-C₆-Alkinyloxy, C₃-C₆-Alkinyloxy-C₁-C₃-alkyl, C₃-C₆-Halogenalkyl, C₃-C₆-Halogenalkenyl, C₃-C₆-Halogenalkinyl, C₃-C₆-Halogenalkoxy, C₃-C₆-Halogenalkoxy-C₁-C₃-alkyl, C₃-C₆-Halogenalkenyloxy, C₃-C₆-Halogenalkenyloxy-C₁-C₃-alkyl, C₃-C₆-Halogenalkinyloxy, C₃-C₆-Halogenalkinyloxy-C₁-C₃-alkyl, C₃-C₆-Alkylthio, C₃-C₆-Alkylthio-C₁-C₃-alkyl, C₂-C₆-Alkylthio-C₂-C₃-alkoxy oder C₂-C₆-Alkylthio-C₂-C₃-alkylthio;
   insbesondere C₄-C₆-Alkyl, C₄-C₆-Cycloalkyl, C₃-C₆-Alkenyl, C₃-C₅-Alkinyl, C₄-C₈-Alkoxy, C₅-C₆-Cycloalkoxy, C₂-C₄-Alkoxy-C₁-C₄-alkyl, C₁-C₃-Alkoxy-C₂-C₄-alkoxy, C₅-C₆-Cycloalkoxy-C₁-C₂-alkyl, C₄-C₆-Alkenyloxy, C₃-C₅-Alkenyloxy-C₁-C₂-alkyl, C₃-C₅-Alkinyloxy, C₃-C₅-Alkinyloxy-C₁-C₂-alkyl, C₃-C₅-Halogenalkyl, C₃-C₅-Halogenalkenyl, C₃-C₅-Halogenalkinyl, C₃-C₅-Halogenalkoxy, C₃-C₅-Halogenalkoxy-C₁-C₂-alkyl, C₃-C₄-Halogenalkenyloxy, C₃-C₄-Halogenalkenyloxy-C₁-C₂-alkyl, C₃-C₄-Halogenalkinyloxy, C₃-C₄-Halogenalkinyloxy-C₁-C₂-alkyl, C₃-C₅-Alkylthio, C₃-C₅-Alkylthio-C₁-C₂-alkyl, C₂-C₄-Alkylthio-C₂-C₃-alkoxy oder C₂-C₄-Alkylthio-C₂-C₃-alkylthio;
   ganz besonders C₄-C₆-Alkyl, Cyclohexyl, C₄-C₅-Alkinyl, C₄-C₅-Alkoxy, C₅-C₆-Cycloalkoxy, C₂-C₄-Alkoxy-C₁-C₄-alkyl, C₁-C₂-Alkoxyethoxy, C₅-C₆-Cycloalkoxymethyl, C₄-C₆-Alkenyloxy, C₃-C₅-Alkenyloxymethyl, C₃-C₅-Alkinyloxy, C₃-C₄-Halogenalkoxy, C₃-Halogenalkenyloxy, C₃-Halogenalkenyloxymethyl, C₃-Halogenalkinyloxy, C₃-Halogenalkinyloxymethyl, C₄-Alkylthio, C₄-Alkylthiomethyl, C₂-C₃-Alkylthioethoxy oder C₂-C₃-Alkylthioethylthio bedeutet;
(2) Eine Verbindung der Formel I, worin
   - R₃: H, Brom oder Methyl;
   vor allem H oder Brom;
   insbesondere H bedeutet;
(3) Eine Verbindung der Formel I, worin
   - R₄: H ist;
(4) Eine Verbindung der Formel I, worin
   - R₅: C₁-C₈-Alkyl, C₃-C₆-Cycloalkyl, C₂-C₄-Alkenyl, C₂-C₃-Alkinyl, C₁-C₃-Alkoxy-C₁-C₃-alkyl, C₂-C₄-Cyanalkyl, C₂-C₆-Halogenalkyl, C₂-C₅-Halogenalkenyl, C₂-C₃-Halogenalkinyl, C₁-C₃-Alkoxycarbonyl, Trifluormethylphenyl, Benzyl oder substituiertes Benzyl, wobei die Substituenten aus der Gruppe, bestehend aus C₁-C₃-Alkyl, C₁-C₃-Alkoxy und Chlor ausgewählt sind;
   vor allem C₁-C₈-Alkyl, C₃-C₆-Cycloalkyl, C₂-C₄-Alkenyl, C₂-C₃-Alkinyl, Methoxyethyl, Cyanmethyl, Halogenethyl, Halogenvinyl, Halogenacetylenyl, Trifluormethylphenyl, Benzyl oder substituiertes Benzyl, wobei die Substituenten aus der Gruppe, bestehend aus Methyl, Methoxy und Chlor ausgewählt sind;
   insbesondere C₁-C₈-Alkyl, Cyclopropyl, C₂-C₄-Alkenyl, C₂-C₃-Alkinyl, Methoxyethyl, Cyanmethyl, Fluorethyl, Chlorethyl, Fluorvinyl, Chlorvinyl, Bromvinyl, Iodacetylenyl, Trifluormethylphenyl, Benzyl, Tolyl, Anisyl oder Chlorphenyl bedeutet;
(5) Eine Verbindung der Formel I, worin
   - R₆: H, Pyridyl, Halogenpyridyl, Furyl oder R₅ und R₆ zusammen C₃-C₅-Alkylen;
   insbesondere H, Pyridyl, Chlorpyridyl, Furyl oder R₅ und R₆ zusammen C₃-C₅-Alkylen bedeuten;
(6) Eine Verbindung der Formel I, worin
   - R₁: C₃-C₆-Alkyl, C₃-C₆-Cycloalkyl, C₃-C₆-Alkenyl, C₃-C₆-Alkinyl, C₃-C₈-Alkoxy, C₃-C₆-Cycloalkoxy, C₂-C₆-Alkoxy-C₁-C₄-alkyl, C₁-C₆-Alkoxy-C₂-C₄-alkoxy, C₄-C₆-Cycloalkoxy-C₁-C₃-alkyl, C₃-C₆-Alkenyloxy, C₃-C₆-Alkenyloxy-C₁-C₃-alkyl, C₃-C₆-Alkinyloxy, C₃-C₆-Alkinyloxy-C₁-C₃-alkyl, C₃-C₆-Halogenalkyl, C₃-C₆-Halogenalkenyl, C₃-C₆-Halogenalkinyl, C₃-C₆-Halogenalkoxy, C₃-C₆-Halogenalkoxy-C₁-C₃-alkyl, C₃-C₆-Halogenalkenyloxy, C₃-C₆-Halogenalkenyloxy-C₁-C₃-alkyl, C₃-C₆-Halogenalkinyloxy, C₃-C₆-Halogenalkinyloxy-C₁-C₃-alkyl, C₃-C₆-Alkylthio, C₃-C₆-Alkylthio-C₁-C₃-alkyl, C₂-C₆-Alkylthio-C₂-C₃-alkoxy oder C₂-C₆-Alkylthio-C₂-C₃-alkylthio;
   - R₂: Chlor oder Brom;
   - R₅: C₁-C₈-Alkyl, C₃-C₆-Cycloalkyl, C₂-C₄-Alkenyl, C₂-C₃-Alkinyl, C₁-C₃-Alkoxy-C₁-C₃-alkyl, C₂-C₄-Cyanalkyl, C₂-C₆-Halogenalkyl, C₂-C₅-Halogenalkenyl, C₂-C₃-Halogenalkinyl, C₁-C₃-Alkoxycarbonyl, Trifluormethylphenyl, Benzyl oder substituiertes Benzyl, wobei die Substituenten aus der Gruppe, bestehend aus C₁-C₃-Alkyl, C₁-C₃-Alkoxy und Chlor ausgewählt sind; und
   - R₆: H, Pyridyl, Halogenpyridyl, Furyl oder R₅ und R₆ zusammen C₃-C₅-Alkylen bedeuten;
(7) Eine Verbindung der Formel I, worin
   - R₁: C₄-C₆-Alkyl, C₄-C₆-Cycloalkyl, C₃-C₆-Alkenyl, C₃-C₅-Alkinyl, C₄-C₈-Alkoxy, C₅-C₆-Cycloalkoxy, C₂-C₄-Alkoxy-C₁-C₄-alkyl, C₁-C₃-Alkoxy-C₂-C₄-alkoxy, C₅-C₆-Cycloalkoxy-C₁-C₂-alkyl, C₄-C₆-Alkenyloxy, C₃-C₅-Alkenyloxy-C₁-C₂-alkyl, C₃-C₅-Alkinyloxy, C₃-C₅-Alkinyloxy-C₁-C₂-alkyl, C₃-C₅-Halogenalkyl, C₃-C₅-Halogenalkenyl, C₃-C₅-Halogenalkinyl, C₃-C₅-Halogenalkoxy, C₃-C₅-Halogenalkoxy-C₁-C₂-alkyl, C₃-C₄-Halogenalkenyloxy, C₃-C₄-Halogenalkenyloxy-C₁-C₂-alkyl, C₃-C₄-Halogenalkinyloxy, C₃-C₄-Halogenalkinyloxy-C₁-C₂-alkyl, C₃-C₅-Alkylthio, C₃-C₅-Alkylthio-C₁-C₂-alkyl, C₂-C₄-Alkylthio-C₂-C₃-alkoxy oder C₂-C₄-Alkylthio-C₂-C₃-alkylthio;
   - R₂: Chlor oder Brom;
   - R₅: C₁-C₈-Alkyl, C₃-C₆-Cycloalkyl, C₂-C₄-Alkenyl, C₂-C₃-Alkinyl, C₁-C₃-Alkoxy-C₁-C₃-alkyl, C₂-C₄-Cyanalkyl, C₂-C₆-Halogenalkyl, C₂-C₅-Halogenalkenyl, C₂-C₃-Halogenalkinyl, C₁-C₃-Alkoxycarbonyl, Trifluormethylphenyl, Benzyl oder substituiertes Benzyl, wobei die Substituenten aus der Gruppe, bestehend aus C₁-C₃-Alkyl, C₁-C₃-Alkoxy und Chlor ausgewählt sind; und
   - R₆: H, Pyridyl, Halogenpyridyl, Furyl oder R₅ und R₆ zusammen C₃-C₅-Alkylen bedeuten;
(8) Eine Verbindung der Formel I, worin
   - R₁: C₄-C₆-Alkyl, C₄-C₆-Cycloalkyl, C₃-C₆-Alkenyl, C₃-C₅-Alkinyl, C₄-C₈-Alkoxy, C₅-C₆-Cycloalkoxy, C₂-C₄-Alkoxy-C₁-C₄-alkyl, C₁-C₃-Alkoxy-C₂-C₄-alkoxy, C₅-C₆-Cycloalkoxy-C₁-C₂-alkyl, C₄-C₆-Alkenyloxy, C₃-C₅-Alkenyloxy-C₁-C₂-alkyl, C₃-C₅-Alkinyloxy, C₃-C₅-Alkinyloxy-C₁-C₂-alkyl, C₃-C₅-Halogenalkyl, C₃-C₅-Halogenalkenyl, C₃-C₅-Halogenalkinyl, C₃-C₅-Halogenalkoxy, C₃-C₅-Halogenalkoxy-C₁-C₂-alkyl, C₃-C₄-Halogenalkenyloxy, C₃-C₄-Halogenalkenyloxy-C₁-C₂-alkyl, C₃-C₄-Halogenalkinyloxy, C₃-C₄-Halogenalkinyloxy-C₁-C₂-alkyl, C₃-C₅-Alkylthio, C₃-C₅-Alkylthio-C₁-C₂-alkyl, C₂-C₄-Alkylthio-C₂-C₃-alkoxy oder C₂-C₄-Alkylthio-C₂-C₃-alkylthio;
   - R₂: Chlor oder Brom;
   - R₅: C₁-C₈-Alkyl, C₃-C₆-Cycloalkyl, C₂-C₄-Alkenyl, C₂-C₃-Alkinyl, C₁-C₃-Alkoxy-C₁-C₃-alkyl, C₂-C₄-Cyanalkyl, C₂-C₆-Halogenalkyl, C₂-C₅-Halogenalkenyl, C₂-C₃-Halogenalkinyl, C₁-C₃-Alkoxycarbonyl, Trifluormethylphenyl, Benzyl oder substituiertes Benzyl, wobei die Substituenten aus der Gruppe, bestehend aus C₁-C₃-Alkyl, C₁-C₃-Alkoxy und Chlor ausgewählt sind; und
   - R₆: H, Pyridyl, Halogenpyridyl, Furyl oder R₅ und R₆ zusammen C₃-C₅-Alkylen bedeuten;
(9) Eine Verbindung der Formel I, worin
   - R₁: C₄-C₆-Alkyl, C₄-C₆-Cycloalkyl, C₃-C₆-Alkenyl, C₃-C₅-Alkinyl, C₄-C₈-Alkoxy, C₅-C₆-Cycloalkoxy, C₂-C₄-Alkoxy-C₁-C₄-alkyl, C₁-C₃-Alkoxy-C₂-C₄-alkoxy, C₅-C₆-Cycloalkoxy-C₁-C₂-alkyl, C₄-C₆-Alkenyloxy, C₃-C₅-Alkenyloxy-C₁-C₂-alkyl, C₃-C₅-Alkinyloxy, C₃-C₅-Alkinyloxy-C₁-C₂-alkyl, C₃-C₅-Halogenalkyl, C₃-C₅-Halogenalkenyl, C₃-C₅-Halogenalkinyl, C₃-C₅-Halogenalkoxy, C₃-C₅-Halogenalkoxy-C₁-C₂-alkyl, C₃-C₄-Halogenalkenyloxy, C₃-C₄-Halogenalkenyloxy-C₁-C₂-alkyl, C₃-C₄-Halogenalkinyloxy, C₃-C₄-Halogenalkinyloxy-C₁-C₂-alkyl, C₃-C₅-Alkylthio, C₃-C₅-Alkylthio-C₁-C₂-alkyl, C₂-C₄-Alkylthio-C₂-C₃-alkoxy oder C₂-C₄-Alkylthio-C₂-C₃-alkylthio;
   - R₂: Chlor oder Brom;
   - R₅: C₁-C₈-Alkyl, C₃-C₆-Cycloalkyl, C₂-C₄-Alkenyl, C₂-C₃-Alkinyl, Methoxyethyl, Cyanmethyl, Halogenethyl, Halogenvinyl, Halogenacetylenyl, Trifluormethylphenyl, Benzyl oder substituiertes Benzyl, wobei die Substituenten aus der Gruppe, bestehend aus Methyl, Methoxy und Chlor ausgewählt sind; und
   - R₆: H, Pyridyl, Halogenpyridyl, Furyl oder R₅ und R₆ zusammen C₃-C₅-Alkylen bedeuten;
(10) Eine Verbindung der Formel I, worin
   - R₁: C₄-C₆-Alkyl, C₄-C₆-Cycloalkyl, C₃-C₆-Alkenyl, C₃-C₅-Alkinyl, C₄-C₈-Alkoxy, C₅-C₆-Cycloalkoxy, C₂-C₄-Alkoxy-C₁-C₄-alkyl, C₁-C₃-Alkoxy-C₂-C₄-alkoxy, C₅-C₆-Cycloalkoxy-C₁-C₂-alkyl, C₄-C₆-Alkenyloxy, C₃-C₅-Alkenyloxy-C₁-C₂-alkyl, C₃-C₅-Alkinyloxy, C₃-C₅-Alkinyloxy-C₁-C₂-alkyl, C₃-C₅-Halogenalkyl, C₃-C₅-Halogenalkenyl, C₃-C₅-Halogenalkinyl, C₃-C₅-Halogenalkoxy, C₃-C₅-Halogenalkoxy-C₁-C₂-alkyl, C₃-C₄-Halogenalkenyloxy, C₃-C₄-Halogenalkenyloxy-C₁-C₂-alkyl, C₃-C₄-Halogenalkinyloxy, C₃-C₄-Halogenalkinyloxy-C₁-C₂-alkyl, C₃-C₅-Alkylthio, C₃-C₅-Alkylthio-C₁-C₂-alkyl, C₂-C₄-Alkylthio-C₂-C₃-alkoxy oder C₂-C₄-Alkylthio-C₂-C₃-alkylthio;
   - R₂: Chlor oder Brom;
   - R₅: C₁-C₈-Alkyl, C₃-C₆-Cycloalkyl, C₂-C₄-Alkenyl, C₂-C₃-Alkinyl, Methoxyethyl, Cyanmethyl, Halogenethyl, Halogenvinyl, Halogenacetylenyl, Trifluormethylphenyl, Benzyl oder substituiertes Benzyl, wobei die Substituenten aus der Gruppe, bestehend aus Methyl, Methoxy und Chlor ausgewählt sind; und
   - R₆: H, Pyridyl, Halogenpyridyl, Furyl oder R₅ und R₆ zusammen C₃-C₅-Alkylen bedeuten;
(11) Eine Verbindung der Formel I, worin
   - R₁: C₄-C₆-Alkyl, Cyclohexyl, C₄-C₅-Alkinyl, C₄-C₅-Alkoxy, C₅-C₆-Cycloalkoxy, C₂-C₄-Alkoxy-C₁-C₄-alkyl, C₁-C₂-Alkoxyethoxy, C₅-C₆-Cycloalkoxymethyl, C₄-C₆-Alkenyloxy, C₃-C₅-Alkenyloxymethyl, C₃-C₅-Alkinyloxy, C₃-C₄-Halogenalkoxy, C₃-C₄-Halogenalkenyloxy, C₃-C₄-Halogenalkenyloxymethyl, C₃-C₄-Halogenalkinyloxy, C₃-C₄-Halogenalkinyloxymethyl, C₃-C₄-Alkylthio, C₃-C₄-Alkylthiomethyl, C₂-C₃-Alkylthioethoxy oder C₂-C₃-Alkylthioethylthio;
   - R₂: Chlor oder Brom;
   - R₅: C₁-C₈-Alkyl, C₃-C₆-Cycloalkyl, C₂-C₄-Alkenyl, C₂-C₃-Alkinyl, Methoxyethyl, Cyanmethyl, Halogenethyl, Halogenvinyl, Halogenacetylenyl, Trifluormethylphenyl, Benzyl oder substituiertes Benzyl, wobei die Substituenten aus der Gruppe, bestehend aus Methyl, Methoxy und Chlor ausgewählt sind; und
   - R₆: H, Pyridyl, Halogenpyridyl, Furyl oder R₅ und R₆ zusammen C₃-C₅-Alkylen bedeuten;
(12) Eine Verbindung der Formel I, worin
   - R₁: C₄-C₆-Alkyl, Cyclohexyl, C₄-C₅-Alkinyl, C₄-C₅-Alkoxy, C₅-C₆-Cycloalkoxy, C₂-C₄-Alkoxy-C₁-C₄-alkyl, C₁-C₂-Alkoxyethoxy, C₅-C₆-Cycloalkoxymethyl, C₄-C₆-Alkenyloxy, C₃-C₅-Alkenyloxymethyl, C₃-C₅-Alkinyloxy, C₃-C₄-Halogenalkoxy, C₃-C₄-Halogenalkenyloxy, C₃-C₄-Halogenalkenyloxymethyl, C₃-C₄-Halogenalkinyloxy, C₃-C₄-Halogenalkinyloxymethyl, C₃-C₄-Alkylthio, C₃-C₄-Alkylthiomethyl, C₂-C₃-Alkylthioethoxy oder C₂-C₃-Alkylthioethylthio;
   - R₂: Chlor oder Brom;
   - R₅: C₁-C₈-Alkyl, Cyclopropyl, C₂-C₄-Alkenyl, C₂-C₃-Alkinyl, Methoxyethyl, Cyanmethyl, Fluorethyl, Chlorethyl, Fluorvinyl, Chlorvinyl, Bromvinyl, Iodacetylenyl, Trifluormethylphenyl, Benzyl, Tolyl, Anisyl oder Chlorphenyl; und
   - R₆: H, Pyridyl, Halogenpyridyl, Furyl oder R₅ und R₆ zusammen C₃-C₅-Alkylen bedeuten;
(13) Eine Verbindung der Formel I, worin
   - R₁: C₄-C₆-Alkyl, Cyclohexyl, C₄-C₅-Alkinyl, C₄-C₅-Alkoxy, C₅-C₆-Cycloalkoxy, C₂-C₄-Alkoxy-C₁-C₄-alkyl, C₁-C₂-Alkoxyethoxy, C₅-C₆-Cycloalkoxymethyl, C₄-C₆-Alkenyloxy, C₃-C₅-Alkenyloxymethyl, C₃-C₅-Alkinyloxy, C₃-C₄-Halogenalkoxy, C₃-C₄-Halogenalkenyloxy, C₃-C₄-Halogenalkenyloxymethyl, C₃-C₄-Halogenalkinyloxy, C₃-C₄-Halogenalkinyloxymethyl, C₃-C₄-Alkylthio, C₃-C₄-Alkylthiomethyl, C₂-C₃-Alkylthioethoxy oder C₂-C₃-Alkylthioethylthio;
   - R₂: Chlor oder Brom;
   - R₅: C₁-C₈-Alkyl, Cyclopropyl, C₂-C₄-Alkenyl, C₂-C₃-Alkinyl, Methoxyethyl, Cyanmethyl, Fluorethyl, Chlorethyl, Fluorvinyl, Chlorvinyl, Bromvinyl, Iodacetylenyl, Trifluormethylphenyl, Benzyl, Tolyl, Anisyl oder Chlorphenyl; und
   - R₆: H, Pyridyl, Chlorpyridyl, Furyl oder R₅ und R₆ zusammen C₃-C₅-Alkylen bedeuten.

Besonders bevorzugt sind im Rahmen der Erfindung die in den Beispielen H4, H8 und H11 aufgeführten Verbindungen der Formel I.

Ganz besonders bevorzugt sind im Rahmen der Erfindung Verbindungen der Formel I, worin R₁ sec-Butoxy oder iso-Butoxy, R₂ Chlor oder Brom, R₃ H oder Brom, R₄ und R₆ H, und R₅ Ethyl oder Propyl bedeuten.

Als weiterer Gegenstand der Erfindung ist das Verfahren zur Herstellung der Verbindungen der Formel I z. B. dadurch gekennzeichnet, dass man
a) eine Verbindung der Formel 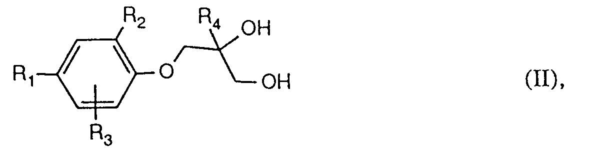 die bekannt ist oder in Analogie zu entsprechenden bekannten Verbindungen hergestellt werden kann und worin R₁, R₂, R₃ und R₄ die für die Formel I angegebenen Bedeutungen haben, in freier Form oder in Salzform, eventuell in Gegenwart eines Säurekatalysators oder eines wasserbindenden Mittels, mit einer Verbindung der Formel

   R₅COR₆ (III),

   die bekannt ist oder in Analogie zu entsprechenden bekannten Verbindungen hergestellt werden kann und worin R₅ und R₆ die für die Formel I angegebenen Bedeutungen haben, oder mit einer Verbindung der Formel 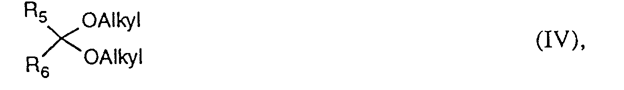 die bekannt ist oder in Analogie zu entsprechenden bekannten Verbindungen hergestellt werden kann und worin R₅ und R₆ die für die Formel I angegebenen Bedeutungen haben und Alkyl Methyl oder Ethyl ist, oder
b) eine Verbindung der Formel 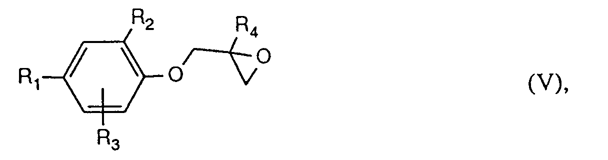 die bekannt ist oder in Analogie zu entsprechenden bekannten Verbindungen hergestellt werden kann und worin R₁, R₂, R₃ und R₄ die für die Formel I angegebenen Bedeutungen haben, eventuell in Gegenwart eines Säurekatalysators oder eines wasserbindenden Mittels, mit einer Verbindung der Formel III, oder
c) eine Verbindung der Formel 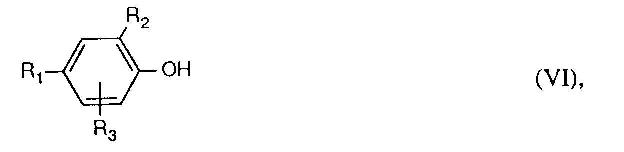 die bekannt ist oder in Analogie zu entsprechenden bekannten Verbindungen hergestellt werden kann und worin R₁, R₂ und R₃ die für die Formel I angegebenen Bedeutungen haben, eventuell in Gegenwart einer Base oder eines wasserbindenden Mittels, mit einer Verbindung der Formel 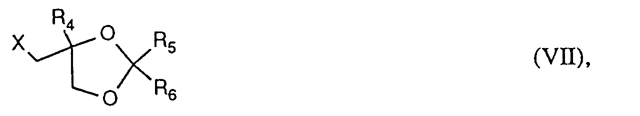 die bekannt ist oder in Analogie zu entsprechenden bekannten Verbindungen hergestellt werden kann und worin R₄, R₅ und R₆ die für die Formel I angegebenen Bedeutungen haben und X eine Abgangsgruppe ist, umsetzt
   und/oder, wenn erwünscht, eine verfahrensgemäss oder auf andere Weise erhältliche Verbindung der Formel I in eine andere Verbindung der Formel I überführt und/oder ein verfahrensgemäss erhältliches Gemisch von Isomeren auftrennt und das gewünschte Isomere isoliert.
   Als weiterer Gegenstand der Erfindung ist das Verfahren zur Herstellung der Verbindungen der Formel II, in freier Form oder in Salzform, z. B. dadurch gekennzeichnet, dass man
d) eine Verbindung der Formel V eventuell in Gegenwart eines Säurekatalysators mit Wasser, oder
e) eine Verbindung der Formel VI, eventuell in Gegenwart einer Base oder eines wasserbindenden Mittels, mit einer Verbindung der Formel 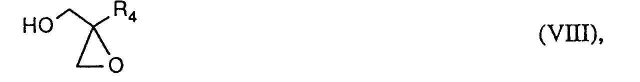 die bekannt ist oder in Analogie zu entsprechenden bekannten Verbindungen hergestellt werden kann und worin R₄ die für die Formel I angegebenen Bedeutungen hat, oder mit einer Verbindung der Formel 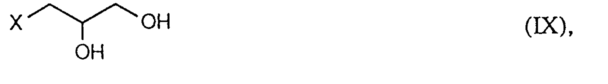 die bekannt ist oder in Analogie zu entsprechenden bekannten Verbindungen hergestellt werden kann und worin X Halogen, Methylsulfonyl oder Toluolsulfonyl, bevorzugt Chlor oder Brom, bedeutet, umsetzt
   und/oder, wenn erwünscht, eine verfahrensgemäss oder auf andere Weise erhältliche Verbindung der Formel II, in freier Form oder in Salzform, in eine andere Verbindung der Formel II überführt, ein verfahrensgemäss erhältliches Gemisch von Isomeren auftrennt und das gewünschte Isomere isoliert und/oder eine verfahrensgemäss oder auf andere Weise erhältliche freie Verbindung der Formel II in ein Salz oder ein verfahrensgemäss oder auf andere Weise erhältliches Salz einer Verbindung der Formel II in die freie Verbindung der Formel II oder in ein anderes Salz überführt.
   Als weiterer Gegenstand der Erfindung ist das Verfahren zur Herstellung der Verbindungen der Formel V z. B. dadurch gekennzeichnet, dass man
f) eine Verbindung der Formel VI, eventuell in Gegenwart eines Basenkatalysators, mit einer Verbindung der Formel 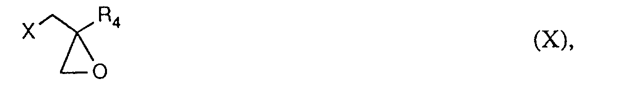 die bekannt ist oder in Analogie zu entsprechenden bekannten Verbindungen hergestellt werden kann und worin R₄ die für die Formel I angegebenen Bedeutungen hat und X Halogen, Methylsulfonyl oder Toluolsulfonyl, bevorzugt Chlor oder Brom, insbesondere Chlor, bedeutet, oder
g) eine verbindung der Formel 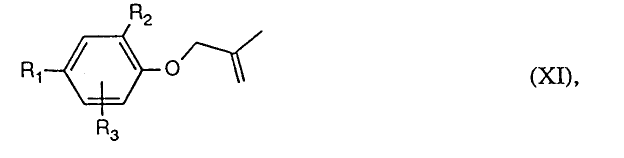 die bekannt ist oder in Analogie zu entsprechenden bekannten Verbindungen hergestellt werden kann und worin R₁, R₂ und R₃ die für die Formel I angegebenen Bedeutungen haben, mit einem Oxidationsmittel umsetzt
   und/oder, wenn erwünscht, eine verfahrensgemäss oder auf andere Weise erhältliche Verbindung der Formel V in eine andere Verbindung der Formel V überführt und/oder ein verfahrensgemäss erhältliches Gemisch von Isomeren auftrennt und das gewünschte Isomere isoliert.

Für vor- und nachstehend aufgeführte Ausgangsmaterialien gilt im Hinblick auf deren Tautomere und/oder Salze das vorstehend für Tautomere und/oder Salze von Verbindungen I, II und V Gesagte in analoger Weise.

Die vor- und nachstehend beschriebenen Umsetzungen werden in an sich bekannter Weise durchgeführt, z. B. in Ab- oder gegebenenfalls in Anwesenheit eines geeigneten Lösungs-oder Verdünnungsmittels oder eines Gemisches derselben, wobei man je nach Bedarf unter Kühlen, bei Raumtemperatur oder unter Erwärmen, z. B. in einem Temperaturbereich von etwa -80°C bis zur Siedetemperatur des Reaktionsgemisches, vorzugsweise von etwa -20°C bis etwa +150°C, und, falls erforderlich, in einem geschlossenen Gefäss, unter Druck, in einer Inertgasatmosphäre und/oder unter wasserfreien Bedingungen arbeitet. Besonders vorteilhafte Reaktionsbedingungen können den Beispielen entnommen werden.

Die vor- und nachstehend aufgeführten Ausgangsmaterialien, die für die Herstellung der Verbindungen I, II und V, in freier Form oder in Salzform, verwendet werden, sind bekannt oder können nach an sich bekannten Methoden, z. B. gemäss den nachstehenden Angaben, hergestellt werden.

### Variante a):

Geeignete Säurekatalysatoren zur Erleichterung der Umsetzung sind z. B. Sulfonsäuren, wie Methan- oder p-Toluolsulfonsäure, Campher-10-Sulfonsäure, Pyridinio-p-toluolsulfonat, einschliesslich der sauren Ionenaustauscherharze mit Sulfogruppen, Lewis-Säuren, wie Bortrifluorid-Diethylether- oder -Dimethylether-Komplexe sowie Mineralsäuren, wie Schwefelsäure oder Phosphorsäure.

Geeignete wasserbindende Mittel zur Erleichterung der Wasser-Abspaltung sind z. B. Carbodiimide, wie N,N'-Dicyclohexylcarbodiimid, oder 1-Alkyl-2-halogen-pyridiniumsalze, wie 1-Methyl-2-chlor-pyridiniumiodid.

Die Reaktionspartner können als solche, d. h. ohne Zusatz eines Lösungs-oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethen; Ether, wie Diethylether, tert.-Butylmethylether, Tetrahydrofuran oder Dioxan; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid.

Die Umsetzung erfolgt vorteilhaft in einem Temperaturbereich von etwa 20°C bis etwa +150°C, bevorzugt von etwa +40°C bis etwa +130°C, in vielen Fällen bei der Rückflusstemperatur des verwendeten Lösungsmittels.

In einer bevorzugten Ausführungsform der Variante a) wird eine Verbindung II bei Rückflusstemperatur, in einem aromatischen Kohlenwasserstoff, vorzugsweise in Toluol, und in Gegenwart einer Sulfonsäre als Katalysator, vorzugsweise in Gegenwart von p-Toluolsulfonsäure, mit einer Verbindung III oder IV umgesetzt, wobei die Verbindungen III oder IV auch im Ueberschuss eingesetzt werden können.

Die Verbindungen der Formel III sind bekannt oder können in Analogie zu bekannten Verbindungen hergestellt werden.

Die Verbindungen IV sind bekannt oder können in Analogie zu bekannten Verbindungen hergestellt werden.

### Variante b):

Geeigneter Säurekatalysator zur Erleichterung der Umsetzung
ist z. B. saure Tonerde, insbesondere Montmorillonit.

Die Reaktionspartner können als solche, d. h. ohne Zusatz eines Lösungs-oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethen; Ether, wie Diethylether, tert.-Butylmethylether, Tetrahydrofuran oder Dioxan; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid.

Die Umsetzung erfolgt vorteilhaft in einem Temperaturbereich von etwa 0°C bis etwa +150°C, bevorzugt von etwa +20°C bis etwa +100°C, in vielen Fällen bei der Rückflusstemperatur des verwendeten Lösungsmittels.

Die Verbindungen der Formel III sind bekannt oder können in Analogie zu bekannten Verbindungen hergestellt werden.

### Variante c):

Geeignete Abgangsgruppen X in Verbindung VII sind z. B. C₁-C₈-Alkansulfonyloxy, Halogen-C₁-C₈-alkansulfonyloxy, Benzolsulfonyloxy, Toluolsulfonyloxy und Halogen. Bevorzugt sind C₁-C₈-Alkoxy und Halogen, insbesondere Chlor und Brom, ganz besonders Chlor.

Geeignete Basen zur Erleichterung der Reaktion sind z.B. Alkalimetall-oder Erdalkalimetall-hydroxide, -hydride, -amide, -alkanolate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natrium-hydroxid, -hydrid, -amid, - methanolat, -carbonat, Kalium-tert.-butanolat, -carbonat, Lithiumdiisopropylamid, Kalium-bis-(trimethylsilyl)-amid, Calciumhydrid, Trimethylamin, Triethylendiamin, Cyclohexylamin, N-Cyclohexyl-N,N-dimethyl-amin, N,N-Diethylanilin, Pyridin, 4-(N,N-Dimethylamino)-pyridin, N-Methylmorpholin, Tetramethylammoniumhydroxid, Benzyltrimethylammoniumhydroxid sowie 1,5-Diazabicyclo[5.4.0]undec-5-en (DBU) genannt. Besonders geeignet sind Alkalimetallcarbonate.

Die Reaktionspartner können als solche, d. h. ohne Zusatz eines Lösungs-oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethen; Ether, wie Diethylether, tert.-Butylmethylether, Tetrahydrofuran oder Dioxan; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid.

Die Umsetzung erfolgt vorteilhaft in einem Temperaturbereich von etwa 20°C bis etwa +150°C, bevorzugt von etwa +50°C bis etwa +100°C, in vielen Fällen bei der Rückflusstemperatur des verwendeten Lösungsmittels.

Die Verbindungen der Formel VII sind bekannt oder können in Analogie zu bekannten Verbindungen hergestellt werden.

### Variante d):

Geeignete Säurekatalysatoren zur Erleichterung der Umsetzung sind z. B. Sulfonsäuren, wie Methan- oder p-Toluolsulfonsäure, Campher-10-Sulfonsäure, Pyridinio-p-toluolsulfonat, einschliesslich der sauren Ionenaustauscherharze mit Sulfogruppen, Lewis-Säuren, wie Bortrifluorid-Diethylether- oder -Dimethylether-Komplexe sowie insbesondere Mineralsäuren, wie Schwefelsäure oder Phosphorsäure.

Die Reaktion kann in Wasser oder bevorzugt in Wasser/Alkohol- oder Wasser/Ether-Gemischen, insbesondere in Wasser/Tetrahydrofuran-Gemischen durchgeführt werden.

Die Umsetzung erfolgt vorteilhaft in einem pH-Bereich des Reaktionsgemisches von 0 bis 5, bevorzugt 1 bis 4, besonders bevorzugt 2 bis 3.

Die Umsetzung erfolgt vorteilhaft in einem Temperaturbereich von etwa 0°C bis etwa +150°C, bevorzugt von etwa +20°C bis etwa +100°C, in vielen Fällen bei der Rückflusstemperatur des verwendeten Lösungsmittels.

### Variante e):

Geeignete Basen zur Erleichterung der Reaktion sind z.B. Alkalimetall-oder Erdalkalimetall-hydroxide, -hydride, -amide, -alkanolate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natrium-hydroxid, -hydrid, -amid, - methanolat, -carbonat, Kalium-tert.-butanolat, -carbonat, Lithiumdiisopropylamid, Kalium-bis-(trimethylsilyl)-amid, Calciumhydrid, Trimethylamin, Triethylendiamin, Cyclohexylamin, N-Cyclohexyl-N,N-dimethyl-amin, N,N-Diethylanilin, Pyridin, 4-(N,N-Dimethylamino)-pyridin, N-Methylmorpholin, Tetramethylammoniumhydroxid, Benzyltrimethylammoniumhydroxid sowie 1,5-Diazabicyclo[5.4.0]undec-5-en (DBU) genannt. Besonders geeignet sind Ammoniumhydroxide oder Alkalimetallcarbonate, ganz besonders Tetramethylammoniumhydroxid oder Kaliumcarbonat.

Die Reaktionspartner können als solche, d. h. ohne Zusatz eines Lösungs-oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethen; Ether, wie Diethylether, tert.-Butylmethylether, Tetrahydrofuran oder Dioxan; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid.

Die Umsetzung erfolgt vorteilhaft in einem Temperaturbereich von etwa 20°C bis etwa +150°C, bevorzugt von etwa +50°C bis etwa +100°C, in vielen Fällen bei der Rückflusstemperatur des verwendeten Lösungsmittels.

Die Verbindungen der Formel VIII sind bekannt oder können in Analogie zu bekannten Verbindungen hergestellt werden.

Die Verbindungen der Formel IX sind bekannt oder können in Analogie zu bekannten Verbindungen hergestellt werden.

### Variante f):

Geeignete Basen zur Erleichterung der Reaktion sind z.B. Alkalimetall-oder Erdalkalimetall-hydroxide, -hydride, -amide, -alkanolate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natrium-hydroxid, -hydrid, -amid, - methanolat, -carbonat, Kalium-tert.-butanolat, -carbonat, Lithiumdiisopropylamid, Kalium-bis-(trimethylsilyl)-amid, Calciumhydrid, Trimethylamin, Triethylendiamin, Cyclohexylamin, N-Cyclohexyl-N,N-dimethyl-amin, N,N-Diethylanilin, Pyridin, 4-(N,N-Dimethylamino)-pyridin, N-Methylmorpholin, Tetramethylammoniumhydroxid, Benzyltrimethylammoniumhydroxid sowie 1,5-Diazabicyclo[5.4.0]undec-5-en (DBU) genannt. Besonders geeignet sind Alkalimetallcarbonate, ganz besonders Kaliumcarbonat.

Die Reaktionspartner können als solche, d. h. ohne Zusatz eines Lösungs-oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethen; Ether, wie Diethylether, tert.-Butylmethylether, Tetrahydrofuran oder Dioxan; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid.

Die Umsetzung erfolgt vorteilhaft in einem Temperaturbereich von etwa 40°C bis etwa +180°C, bevorzugt von etwa +60°C bis etwa +150°C, in vielen Fällen bei der Rückflusstemperatur des verwendeten Lösungsmittels.

Die Reaktionspartner können in molaren Mengen, bevorzugt im Ueberschuss der Verbindung X, miteinander umgesetzt werden.

Die Verbindungen der Formel X sind bekannt oder können in Analogie zu bekannten Verbindungen hergestellt werden.

### Variante g):

Geeignete Oxidationsmittel sind z. B. organische Percarbonsäuren, wie Peressigsäure, Perbenzoesäure oder vorzugsweise substituierte Perbenzoesäuren, besonders 3-Chlorperbenzoesäure.

Die Reaktionspartner können als solche, d. h. ohne Zusatz eines Lösungs-oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethen; Ether, wie Diethylether, tert.-Butylmethylether, Tetrahydrofuran oder Dioxan; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid.

Die Umsetzung erfolgt vorteilhaft in einem Temperaturbereich von etwa 20°C bis etwa +150°C, bevorzugt von etwa +40°C bis etwa +130°C, in vielen Fällen bei der Rückflusstemperatur des verwendeten Lösungsmittels.

Die Verbindungen I, II und V können in Form eines der möglichen Isomeren oder als Gemisch derselben, z. B. je nach Anzahl, absoluter und relativer Konfiguration der asymmetrischen Kohlenstoffatome als reine Isomere, wie Antipoden und/oder Diastereomere, oder als Isomerengemische, wie Enantiomerengemische, z. B. Racemate, Diastereomerengemische oder Racematgemische, vorliegen; die Erfindung betrifft sowohl die reinen Isomeren als auch alle möglichen Isomerengemische und ist vor-und nachstehend jeweils entsprechend zu verstehen, auch wenn stereochemische Einzelheiten nicht in jedem Fall speziell erwähnt werden.

Verfahrensgemäss - je nach Wahl der Ausgangsstoffe und Arbeitsweisen - oder anderweitig erhältliche Diastereomerengemische und Racematgemische von Verbindungen I, II und V können auf Grund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Entsprechend erhältliche Enantiomerengemische, wie Racemate, lassen sich nach bekannten Methoden in die optischen Antipoden zerlegen, beispielsweise durch Umkristallisation aus einem optisch aktiven Lösungsmittel, durch Chromatographie an chiralen Adsorbentien, z. B. Hochdruckflüssigkeitschromatographie (HPLC) an Acetylcellulose, mit Hilfe von geeigneten Mikroorganismen, durch Spaltung mit spezifischen, immobilisierten Enzymen, über die Bildung von Einschlussverbindungen, z. B. unter Verwendung chiraler Kronenether, wobei nur ein Enantiomeres komplexiert wird.

Ausser durch Auftrennung entsprechender Isomerengemische können reine Diastereomere bzw. Enantiomere erfindungsgemäss auch durch allgemein bekannte Methoden der diastereoselektiven bzw. enantioselektiven Synthese erhalten werden, z. B. indem man das erfindungsgemässe Verfahren mit Edukten mit entsprechend geeigneter Stereochemie ausführt.

Vorteilhaft isoliert bzw. synthetisiert man jeweils das biologisch wirksamere Isomere, z. B. Enantiomere, oder Isomerengemisch, z. B. Enantiomerengemisch, sofern die einzelnen Komponenten unterschiedliche biologische Wirksamkeit besitzen.

Die Verbindungen I, II und V können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Erfindung betrifft alle diejenigen Ausführungsformen des Verfahrens, nach denen man von einer auf irgendeiner Stufe des Verfahrens als Ausgangs- oder Zwischenprodukt erhältlichen Verbindung ausgeht und alle oder einige der fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe und Zwischenprodukte verwendet, welche zu den eingangs als besonders wertvoll geschilderten Verbindungen I führen.

Die Erfindung betrifft insbesondere die in den Beispielen H1 bis H5 beschriebenen Herstellungsverfahren.

Erfindungsgemäss für die Herstellung der Verbindungen I verwendete Ausgangsstoffe und Zwischenprodukte, die neu sind, ihre Verwendung und Verfahren zu ihrer Herstellung bilden ebenfalls einen Gegenstand der Erfindung.

In diesem Zusammenhang sind die Verbindungen II und V von spezieller Bedeutung, welche einen weiteren Gegenstand der vorliegenden Erfindung bilden, ebenso wie ihre Herstellung und ihre Verwendung als Zwischenprodukte.

Die erfindungsgemässen Verbindungen I sind auf dem Gebiet der Schädlingsbekämpfung bei günsliger Warmblüter-, Fisch- und Pflanzenverträglichkeit bereits bei niedrigen Anwendungskonzentrationen präventiv und/oder kurativ wertvolle Wirkstoffe mit einem sehr günstigen bioziden Spektrum. Die erfindungsgemässen Wirkstoffe sind gegen alle oder einzelne Entwicklungsstadien von normal sensiblen, aber auch von resistenten, tierischen Schädlingen, wie Insekten oder Vertetern der Ordnung Acarina, wirksam. Die insektizide oder akarizide Wirkung der erfindungsgemässen Wirkstoffe kann sich dabei direkt, d. h. in einer Abtötung der Schädlinge, welche unmittelbar oder erst nach einiger Zeit, beispielsweise bei einer Häutung, eintritt, oder indirekt, z. B. in einer verminderten Eiablage und/oder Schlupfrate, zeigen, wobei die gute Wirkung einer Abtötungsrate (Mortalität) von mindestens 50 bis 60% entspricht.

Zu den erwähnten tierischen Schädlingen gehören beispielsweise:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung Orthoptera zum Beispiel
Blatta spp., Blattella spp., Gryllotalpa spp., Leucophaea maderae, Locusta spp., Periplaneta spp. und Schistocerca spp.;
aus der Ordnung Isoptera zum Beispiel
Reticulitermes spp.;
aus der Ordnung Psocoptera zum Beispiel
Liposcelis spp.;
aus der Ordnung Anoplura zum Beispiel
Haematopinus spp., Linognathus spp., Pediculus spp., Pemphigus spp. und Phylloxera spp.;
aus der Ordnung Mallophaga zum Beispiel
Damalinea spp. und Trichodectes spp.;
aus der Ordnung Thysanoptera zum Beispiel
Frankliniella spp., Hercinothrips spp., Taeniothrips spp., Thrips palmi, Thrips tabaci und Scirtothrips aurantii;
aus der Ordnung Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung Siphonaptera zum Beispiel
Ceratophyllus spp. und Xenopsylla cheopis;
aus der Ordnung Thysanura zum Beispiel
Lepisma saccharina und
aus der Ordnung Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp..

Mit den erfindungsgemässen Wirkstoffen kann man insbesondere an Pflanzen, vor allem an Nutz- und Zierpflanzen in der Landwirtschaft, im Gartenbau und im Forst, oder an Teilen, wie Früchten, Blüten, Laubwerk, Stengeln, Knollen oder Wurzeln, solcher Pflanzen auftretende Schädlinge des erwähnten Typus bekämpfen, d. h. eindämmen oder vernichten, wobei zum Teil auch später zuwachsende Pflanzenteile noch gegen diese Schädlinge geschützt werden.

Als Zielkulturen kommen insbesondere Getreide, wie Weizen, Gerste, Roggen, Hafer, Reis, Mais oder Sorghum; Rüben, wie Zucker- oder Futterrüben; Obst, z. B. Kern-, Stein-und Beerenobst, wie Aepfel, Birnen, Pflaumen, Pfirsiche, Mandeln, Kirschen oder Beeren, z. B. Erdbeeren, Himbeeren oder Brombeeren; Hülsenfrüchte, wie Bohnen, Linsen, Erbsen oder Soja; Oelfrüchte, wie Raps, Senf, Mohn, Oliven, Sonnenblumen, Kokos, Rizinus, Kakao oder Erdnüsse; Gurkengewächse, wie Kürbisse, Gurken oder Melonen; Fasergewächse, wie Baumwolle, Flachs, Hanf oder Jute; Citrusfrüchte, wie Orangen, Zitronen, Pampelmusen oder Mandarinen; Gemüse, wie Spinat, Kopfsalat, Spargel, Kohlarten, Möhren, Zwiebeln, Tomaten, Kartoffeln oder Paprika; Lorbeergewächse, wie Avocado, Cinnamonium oder Kampfer; sowie Tabak, Nüsse, Kaffee, Eierfrüchte, Zuckerrohr, Tee, Pfeffer, Weinreben, Hopfen, Bananengewächse, Naturkautschukgewächse und Zierpflanzen in Betracht.

Weitere Anwendungsgebiete der erfindungsgemässen Wirkstoffe sind der Schutz von Vorräten und Lagern und von Material sowie im Hygienesektor insbesondere der Schutz von Haus- und Nutztieren vor Schädlingen des erwähnten Typus.

Die Erfindung betrifft daher auch Schädlingsbekämpfungsmittel, wie, je nach angestrebten Zielen und gegebenen Verhältnissen zu wählende, emulgierbare Konzentrate, Suspensionskonzentrate, direkt versprüh- oder verdünnbare Lösungen, streichfähige Pasten, verdünnte Emulsionen, Spritzpulver, lösliche Pulver, dispergierbare Pulver, benetzbare Pulver, Stäubemittel, Granulate oder Verkapselungen in polymeren Stoffen, welche - mindestens - einen der erfindungsgemässen Wirkstoffe enthalten.

Der Wirkstoff wird in diesen Mitteln in reiner Form, ein fester Wirkstoff z. B. in einer speziellen Korngrösse, oder vorzugsweise zusammen mit - mindestens - einem der in der Formulierungstechnik üblichen Hilfsstoffe, wie Streckmitteln, z. B. Lösungsmitteln oder festen Trägerstoffen, oder wie oberflächenaktiven Verbindungen (Tensiden), eingesetzt.

Als Lösungsmittel können z. B. in Frage kommen: gegebenenfalls partiell hydrierte aromatische Kohlenwasserstoffe, bevorzugt die Fraktionen C₈ bis C₁₂ von Alkylbenzolen, wie Xylolgemische, alkylierte Naphthaline oder Tetrahydronaphthalin, aliphatische oder cycloaliphatische Kohlenwasserstoffe, wie Paraffine oder Cyclohexan, Alkohole, wie Ethanol, Propanol oder Butanol, Glykole sowie deren Ether und Ester, wie Propylenglykol, Dipropylenglykolether, Ethylenglykol oder Ethylenglykolmono-methyl- oder -ethylether, Ketone, wie Cyclohexanon, Isophoron oder Diacetonalkohol, stark polare Lösungsmittel, wie N-Methylpyrrolid-2-on, Dimethylsulfoxid oder N,N-Dimethylformamid, Wasser, gegebenenfalls epoxidierte Pflanzenöle, wie gegebenenfalls epoxidiertes Raps-, Rizinus-, Kokosnuss- oder Sojaöl, und Silikonöle.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, und als nicht sorptive Trägermaterialien Calcit oder Sand in Frage. Darüberhinaus kann eine Vielzahl von granulierten Materialien anorganischer oder organischer Natur, insbesondere Dolomit oder zerkleinerte Pflanzenrückstände, verwendet werden.

Als oberflächenaktive Verbindungen kommen, je nach Art des zu formulierenden Wirkstoffs, nichtionische, kationische und/oder anionische Tenside oder Tensidgemische mit guten Emulgier-, Dispergier- und Netzeigenschaften in Betracht. Die nachstehend aufgeführten Tenside sind dabei nur als Beispiele anzusehen; in der einschlägigen Literatur werden viele weitere in der Formulierungstechnik gebräuchliche und erfindungsgemäss geeignete Tenside beschrieben.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignet sind wasserlösliche, 20 bis 250 Ethylenglykolether- und 10 bis 100 Propylenglykolether-gruppen enthaltende, Polyethylenoxid-Addukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykol-Einheiten. Als Beispiele seien Nonylphenolpolyethoxyethanole, Ricinusölpolyglykolether, Polypropylen-Polyethylenoxid-Addukte, Tributylphenoxypolyethoxyethanol, Polyethylenglykol und Octylphenoxypolyethoxyethanol erwähnt. Ferner kommen Fettsäureester von Polyoxyethylensorbitan, wie das Polyoxyethylensorbitan-trioleat, in Betracht.

Bei den kationischen Tensiden handelt es sich vor allem um quarternäre Ammoniumsalze, welche als Substituenten mindestens einen Alkylrest mit 8 bis 22 C-Atomen und als weitere Substituenten niedrige, gegebenenfalls halogenierte, Alkyl-, Benzyl-oder niedrige Hydroxyalkylreste aufweisen. Die Salze liegen vorzugsweise als Halogenide, Methylsulfate oder Ethylsulfate vor. Beispiele sind das Stearyl-trimethyl-ammoniumchlorid und das Benzyl-di-(2-chlorethyl)-ethyl-ammoniumbromid.

Geeignete anionische Tenside können sowohl wasserlösliche Seifen als auch wasserlösliche synthetische oberflächenaktive Verbindungen sein. Als Seifen eignen sich die Alkali-, Erdalkali- und gegebenenfalls substituierten Ammoniumsalze von höheren Fettsäuren (C₁₀-C₂₂), wie die Natrium- oder Kalium-Salze der Oel- oder Stearinsäure, oder von natürlichen Fettsäuregemischen, die beispielsweise aus Kokosnuss-oder Tallöl gewonnen werden können; ferner sind auch die Fettsäuremethyl-taurin-salze zu erwähnen. Häufiger werden jedoch synthetische Tenside verwendet, insbesondere Fettsulfonate, Fettsulfate, sulfonierte Benzimidazolderivate oder Alkylarylsulfonate. Die Fettsulfonate und -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst; beispielhaft genannt seien das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit etwa 8 bis 22 C-Atomen. Alkylarylsulfonate sind zum Beispiel die Natrium-, Calcium- oder Triethanolammoniumsalze der Dodecylbenzolsulfonsäure, der Dibutylnaphthalinsulfonsäure oder eines Naphthalinsulfonsäure-Formaldehyd-Kondensationsproduktes. Ferner kommen auch entsprechende Phosphate, wie Salze des Phosphorsäureesters eines p-Nonylphenol-(4-14)-Ethylenoxid-Adduktes oder Phospholipide, in Frage.

Die Mittel enthalten in der Regel 0,1 bis 99%, insbesondere 0,1 bis 95%, Wirkstoff und 1 bis 99,9%, insbesondere 5 bis 99,9%, - mindestens - eines festen oder flüssigen Hilfsstoffes, wobei in der Regel 0 bis 25%, insbesondere 0,1 bis 20%, der Mittel Tenside sein können (% bedeutet jeweils Gewichtsprozent). Während als Handelsware eher konzentrierte Mittel bevorzugt werden, verwendet der Endverbraucher in der Regel verdünnte Mittel, die wesentlich geringere Wirkstoffkonzentrationen aufweisen. Bevorzugte Mittel setzen sich insbesondere folgendermassen zusammen (% = Gewichtsprozent):

| Emulgierbare Konzentrate: | |
|---|---|
| Wirkstoff: | 1 bis 90%, vorzugsweise 5 bis 20% |
| Tensid: | 1 bis 30%, vorzugsweise 10 bis20 % |
| Lösungsmittel: | 5 bis 98%, vorzugsweise 70 bis 85% |

| Stäubemittel: | |
|---|---|
| Wirkstoff: | 0,1 bis 10%, vorzugsweise 0,1 bis 1% |
| fester Trägerstoff: | 99,9 bis 90%, vorzugsweise 99,9 bis 99% |

| Suspensionskonzentrate: | |
|---|---|
| Wirkstoff: | 5 bis 75%, vorzugsweise 10 bis 50% |
| Wasser: | 94 bis 24%, vorzugsweise 88 bis 30% |
| Tensid: | 1 bis 40%, vorzugsweise 2 bis 30% |

| Benetzbare Pulver: | |
|---|---|
| Wirkstoff: | 0,5 bis 90%, vorzugsweise 1 bis 80% |
| Tensid: | 0,5 bis 20%, vorzugsweise 1 bis 15% |
| fester Trägerstoff: | 5 bis 99%, vorzugsweise 15 bis 98% |

| Granulate: | |
|---|---|
| Wirkstoff: | 0,5 bis 30%, vorzugsweise 3 bis 15% |
| fester Trägerstoff: | 99,5 bis 70%, vorzugsweise 97 bis 85% |

Die Wirkung der erfindungsgemässen Mittel lässt sich durch Zusatz von anderen insektiziden oder akariziden Wirkstoffen wesentlich verbreitern und an gegebene Umstände anpassen. Als insektizide oder akarizide Wirkstoff-Zusätze kommen dabei z. B. Vertreter der folgenden Wirkstoffklassen in Betracht: Organische Phosphorverbindungen, Nitrophenole und Derivate, Formamidine, Acylharnstoffe, Carbamate, Pyrethroide, Nitroenamine und Derivate, Pyrrole, Thioharnstoffe und Derivate, chlorierte Kohlenwasserstoffe und Bacillus thuringiensis-Präparate. Die erfindungsgemässen Mittel können auch weitere feste oder flüssige Hilfsstoffe, wie Stabilisatoren, z. B. gegebenenfalls epoxidierte Pflanzenöle (z. B. epoxidiertes Kokosnussöl, Rapsöl oder Sojaöl), Entschäumer, z. B. Silikonöl, Konservierungsmittel, Viskositätsregulatoren, Bindemittel und/oder Haftmittel, sowie Düngemittel oder andere Wirkstoffe zur Erzielung spezieller Effekte, z. B. Bakterizide, Fungizide, Nematozide, Molluskizide oder selektive Herbizide, enthalten.

Die erfindungsgemässen Mittel werden in bekannter Weise hergestellt, bei Abwesenheit von Hilfsstoffen z. B. durch Mahlen, Sieben und/oder Presssen eines festen Wirkstoffs oder Wirkstoffgemisches, z. B. auf eine bestimmte Korngrösse, und bei Anwesenheit von mindestens einem Hilfsstoff z. B. durch inniges Vermischen und/oder Vermahlen des Wirkstoffs oder Wirkstoffgemisches mit dem (den) Hilfsstoff(en). Diese Verfahren zur Herstellung der erfindungsgemässen Mittel und die Verwendung der Verbindungen I zur Herstellung dieser Mittel bilden ebenfalls einen Gegenstand der Erfindung.

Die Anwendungsverfahren für die Mittel, also die Verfahren zur Bekämpfung von Schädlingen des erwähnten Typus, wie, je nach angestrebten Zielen und gegebenen Verhältnissen zu wählendes, Versprühen, Vernebeln, Bestäuben, Bestreichen, Beizen, Streuen oder Giessen, und die Verwendung der Mittel zur Bekämpfung von Schädlingen des erwähnten Typus sind weitere Gegenstände der Erfindung. Typische Anwendungskonzentrationen liegen dabei zwischen 0,1 und 1000 ppm, bevorzugt zwischen 0,1 und 500 ppm, Wirkstoff. Die Aufwandmengen pro Hektar betragen im allgemeinen 1 bis 2000 g Wirkstoff pro Hektar, insbesondere 10 bis 1000 g/ha, vorzugsweise 20 bis 600 g/ha.

Ein bevorzugtes Anwendungsverfahren auf dem Gebiet des Pflanzenschutzes ist das Aufbringen auf das Blattwerk der Pflanzen (Blattapplikation), wobei sich Applikationsfrequenz und Aufwandmenge auf den Befallsdruck des jeweiligen Schädlings ausrichten lassen. Der Wirkstoff kann aber auch durch das Wurzelwerk in die Pflanzen gelangen (systemische Wirkung), indem man den Standort der Pflanzen mit einem flüssigen Mittel tränkt oder den Wirkstoff in fester Form in den Standort der Pflanzen, z. B. in den Boden, einbringt, z. B. in Form von Granulat (Bodenapplikation). Bei Wasserreiskulturen kann man solche Granulate dem überfluteten Reisfeld zudosieren.

Die erfindungsgemässen Mittel eignen sich auch für den Schutz von pflanzlichem Vermehrungsgut, z. B. Saatgut, wie Früchten, Knollen oder Körnern, oder Pflanzenstecklingen, vor tierischen Schädlingen. Das Vermehrungsgut kann dabei vor dem Ausbringen mit dem Mittel behandelt, Saatgut z. B. vor der Aussaat gebeizt, werden. Die erfindungsgemässen Wirkstoffe können auch auf Samenkörner aufgebracht werden (Coating), indem man die Körner entweder in einem flüssigen Mittel tränkt oder sie mit einem festen Mittel beschichtet. Das Mittel kann auch beim Ausbringen des Vermehrungsguts auf den Ort der Ausbringung, z. B. bei der Aussaat in die Saatfurche, appliziert werden. Diese Behandlungsverfahren für pflanzliches Vermehrungsgut und das so behandelte pflanzliche Vermehrungsgut sind weitere Gegenstände der Erfindung.

Die folgenden Beispiele dienen der Erläuterung der Erfindung. Sie schränken die Erfindung nicht ein. Temperaturen sind in Grad Celsius angegeben. "n_{D}^{T}" steht für den Brechungsindex bei einer Temperatur von T°C. "Smp." steht für Schmelzpunkt. "%" bedeutet Gewichtsprozent, sofern nichts anderes angegeben ist.

### Herstellungsbeispiele

### Beispiel H1:

2-Chlor-4-(1-methylpropoxy)-phenol

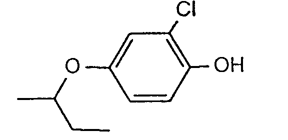

Zu einer Lösung von 91,3 g 4-(1-Methylpropoxy)-phenol in 900 ml Dichlormethan tropft man unter Rühren bei 0° bis 5°C innerhalb von ca. 30 Min. 76,8 g Sulfurylchlorid. Anschliessend wird 14 Std. bei Raumtemperatur weitergerührt. Hierauf wird das Reaktionsgemisch im Vakuum vollständig eingedampft und der Rückstand an Kieselgel chromatographiert (Eluierungsmittel: Diethylether/n-Hexan 1:9), wodurch das reine Produkt mit einem Brechungsindex n_{D}²⁰ von 1,5321 erhalten wird.

In analoger Weise kann man herstellen:

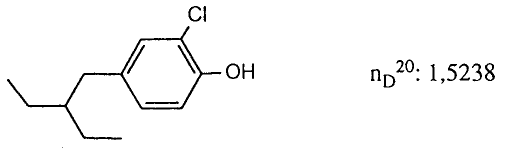

### Beispiel H2:

2,6-Dibrom-4-(1-methylpropoxy)-phenol

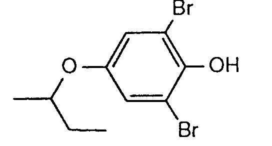

Zu der Lösung von 24,9 g 4-(1-Methylpropoxy)-phenol in 120 ml Dichlormethan werden unter Rühren bei 0° bis 5°C innerhalb von 2 Std. 48 g Brom getropft. Anschliessend wird auf 20-22°C erwärmt und weitere 10 Std. bei dieser Temperatur gerührt. Hierauf wird das Reaktionsgemisch im Vakuum eingedampft und der Rückstand an Kieselgel chromatographisch gereinigt (Eluierungsmittel: Diethylether/n-Hexan 1:5), wodurch das reine Produkt mit einem Brechungsindex n_{D}²⁰ von 1,5780 erhalten wird.

In analoger Weise kann man herstellen:

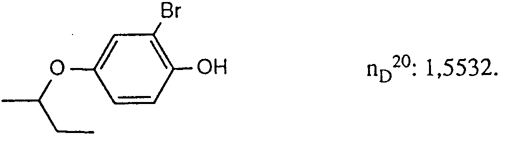

### Beispiel H3:

3-[2-Chlor-4-(1-methylpropoxy)-phenoxy]-1,2-propandiol

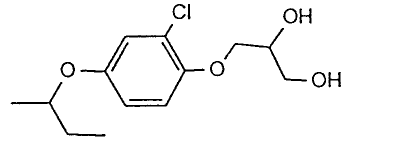

Zu der Lösung von 40,1 g 2-Chlor-4-(1-methylpropoxy)-phenol in 300 ml Xylol gibt man 0,3 g Tetramethylammoniumchlorid, erwärmt auf 60°C und tropft unter Rühren in ca. 30 Min. 16,3 g Glyceringlycid zu. Hierauf wird das Reaktionsgemisch während 10 Std. bei 90°C weitergerührt. Zur Isolierung wird das Lösungsmittel am Rotationsverdampfer im Vakuum abdestilliert und der Rückstand an Kieselgel chromatographiert (Eluierungsmittel: Diethylether/Methylenchlorid 1:2), wodurch das reine Produkt mit einem Brechungsindex n_{D}²⁰ von 1,5330 erhalten wird.

In analoger Weise kann man herstellen:

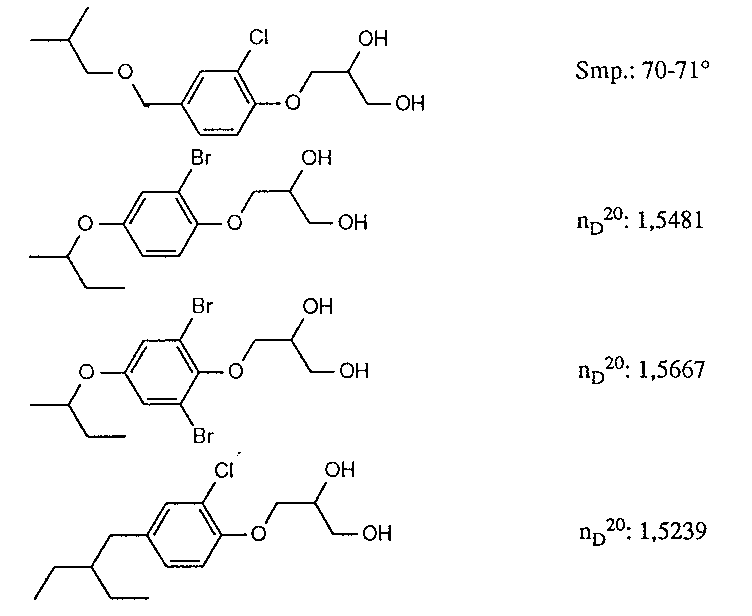

In analoger Weise können auch alle in den Tabellen 5 bis 8 aufgeführten Beispiele hergestellt werden.

### Beispiel H4:

3-Ethyl-4-[2-chlor-4-(1-methylpropoxy)-phenoxymethyl]-1,3-dioxolan

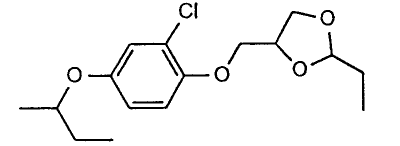

Zu der Lösung von 8,2 g 3-[2-Chlor-4-(1-methylpropoxy)-phenoxy]-1,2-propandiol und 30 mg 4-Toluolsulfonsäure in 80 ml Toluol werden unter Rühren 2,4 g frisch destillierter Propionaldehyd zugefügt und das Reaktionsgemisch 2 Std. bei Rückflusstemperatur gerührt. Hierauf wird das Reaktionsgemisch wiederholt mit 10%iger Natriumcarbonatlösung und anschliessend mit Wasser gewaschen, die Toluollösung über Natriumsulfat getrocknet, das Lösungsmittel im Vakuum vollständig abdestilliert und der Rückstand an Kieselgel chromatographiert (Eluierungsmittel: Diethylether/n-Hexan 1:9), wodurch zwei Diastereoisomere A und B isoliert wurden.

In analoger Weise kann man jeweils zwei Diastereoisomere A und B der folgenden Verbindungen herstellen:

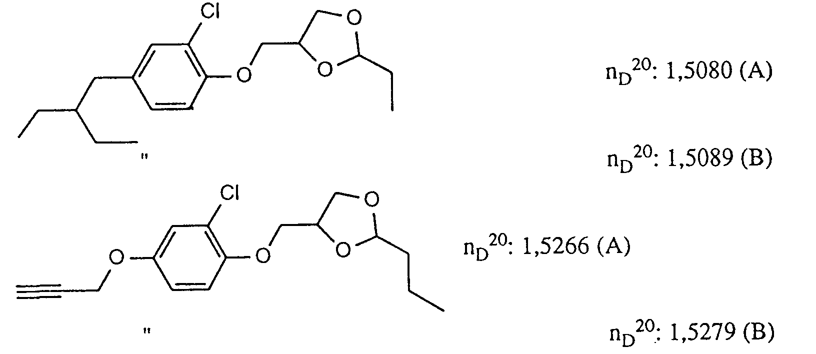

In analoger Weise können auch alle in den Tabellen 1 bis 4 aufgeführten Beispiele hergestellt werden.

### Beispiel H5:

1-Benzyloxy-4-(2-methylpropoxymethyl)-benzol

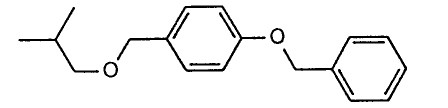

Eine Lösung von 74,9 g 4-Benzyloxybenzylalkohol, 19 g Tetrabutylammoniumhydrogensulfat und 224 g 50%ige Natronlauge in 300 ml Toluol rührt man 15 min bei 40°. Anschliessend tropft man bei dieser Temperatur innert 2 h 192 g Isobutylbromid zu und rührt bei derselben Temperatur weitere 20 h. Danach wird das Reaktionsgemisch auf Eiswasser gegossen, die organische Phase abgetrennt und die wässrige Phase wiederholt mit Toluol extrahiert. Die vereinigten organischen Phasen werden mit Wasser neutral gewaschen, mit Natriumsulfat getrocknet und eingedampft. Nach Reinigung des Rückstandes an Kieselgel mit Diethylether/n-Hexan (1:19) erhält man die reine Titelverbindung mit einem Brechungsindex n_{D}²⁰ von 1,5399.

### Beispiel H6:

4-(2-Methylpropoxymethyl)-phenol

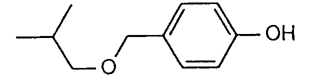

Zu einer Lösung von 108 g 1-Benzyloxy-4-(2-methylpropoxymethyl)-benzol und 216 ml Triethylamin in 870 ml Tetrahydrofuran gibt man 1 g Palladiumkohle (5%Pd) und hydrogenolysiert in einer Hydrierapparatur bei 100 mbar Ueberdruck und Raumtemperatur mit Wasserstoff, wobei innert 4 h 8.93 lt Wasserstoff aufgenommen wird. Nach anschliessender Filtration der Suspension durch Diatomeenerde wird das Lösungsmittel abdestilliert und der Rückstand an Kieselgel mit Diethylether/n-Hexan (1:3) chromatographiert, wobei 69,3 g der Titelverbindung mit einem Brechungsindex n_{D}²⁰ von 1,5139 isoliert werden.

### Beispiel H7:

2-Chlor-4-(2-methylpropoxymethyl)-phenol

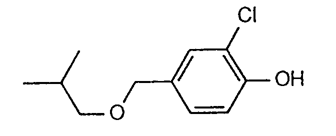

Zu einer Lösung von 59,4 g 4-(2-Methylpropoxymethyl)-phenol in 500 ml Tetrachlorkohlenstoff tropft man unter Rühren bei 0° innert 1 h 35,8 g t-Butylhypochlorit, lässt anschliessend die Temperatur auf 20° ansteigen und rührt weitere 30 min. Nach Entfernung des Lösungsmittels durch Destillation wird der Rückstand in Essigsäureethylester aufgenommen, die Lösung zweimal mit wässriger, 5%iger Natriumhydrogencarbonat-Lösung und zweimal mit Wasser gewaschen. Nach Trocknung der Lösung mit Natriumsulfat und Eindampfen wird der Rückstand auf Kieselgel mit Diethylether/n-Hexan (1:19) chromatographiert, wobei man die reine Titelverbindung mit einem Brechungsindex n_{D}²⁰ von 1,5251 erhält.

### Beispiel H8:

2-Ethyl-4-[2-chlor-4-(2-methylpropoxymethyl)-phenoxymethyl]-1,3-dioxolan (Diastereoisomerengemisch)

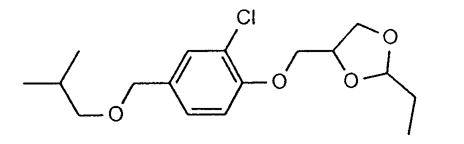

Analog zum Beispiel H4 wird aus 3-[2-Chlor-4-(2-methylpropoxymethyl)-phenoxy-1,2-propandiol und Propionaldehyd die Titelverbindung als Diastereoisomerengemisch erhalten, das sich durch Chromatographie auf Kieselgel mit Diethylether/n-Hexan (1:19) in die Diastereoisomeren A, mit einem Brechungsindex n_{D}²⁰ von 1,5035, und B, mit einem Brechungsindex n_{D}²⁰ von 1,5044, auftrennen lässt.

### Beispiel H9:

4-(2-Ethylpropanoyl)-anisol

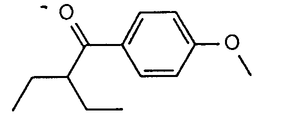

Zu einer Lösung von 21,6 g Anisol in 180 ml Dichlormethan gibt man unter Rühren 32 g Aluminiumtrichlorid, kühlt auf -10° und tropft unter Rühren 28,2 g 2-Ethylbuttersäurechlorid innert 30 min zu. Nach einer weiteren Stunde Rührens bei - 10° wird auf 0° erwärmen lassen und das Reaktionsgemisch in eine Mischung von 30 ml 37%iger Salzsäure und 600 ml Eiswasser gegossen. Die organische Phase wird abgetrennt und die wässrige Phase dreimal mit Dichlormethan extrahiert. Die vereinigten organischen Phasen werden anschliessend mit 1 N Natronlauge und dann wiederholt mit Wasser gewaschen und nach Trocknen mit Natriumsulfat eingedampft. Es werden so 38 g reine Titelverbindung mit einem Brechungsindex n_{D}²⁰ von 1,5226 wird durch Chromatographie auf Kieselgel mit Diethylether/n-Hexan (1:9) erhalten.

### Beispiel H10:

4-(2-Ethylbutyl)-phenol

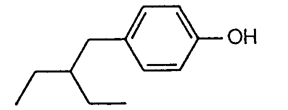

Ein Gemisch aus 16,5 g 4-(2-Ethylpropanoyl)-anisol, 16 g Hydrazinhydrat, 160 ml Triethylenglykol und 18 g fein pulverisiertem Kaliumhydroxid wird unter Rühren in einer Schutzgasatmosphäre langsam auf 190-200° erhitzt und das verdampfende Reaktionswasser und etwas Hydrazinhydrat mit einem absteigenden Kühler kondensiert. Nach 6 h Rührens bei 200° wird das Reaktionsgemisch auf Raumtemperatur gekühlt, mit Wasser versetzt und wiederholt mit t-Butylmethylelher extrahiert. Die vereinigten organischen Phasen werden anschliessend mehrmals mit 1N Salzsäure und dann mit Wasser gewaschen, mit Natriumsulfat getrocknet und eingedampft. Nach chromatographischer Reinigung auf Kieselgel mit Diethylether/n-Hexan (1:9) wird die reine Titelverbindung mit einem Brechungsindex n_{D}²⁰ von 1,5163 erhalten.

### Beispiel H11:

In analoger Weise wie in den Beispielen H4 und H8 beschrieben können auch die anderen in den Tabellen 1 bis 4 aufgeführten Verbindungen hergestellt werden. Die in den Tabellen 5 bis 8 aufgeführten Zwischenprodukte können in analoger Weise wie im Beispiel H3 beschrieben aus bekannten oder aus in Analogie zu bekannten Verbindungen herstellbaren Ausgangsprodukten hergestellt werden. In der Spalte "physik. Daten" dieser Tabellen bedeutet "n_{D}²⁰" den Brechungsindex der betreffenden Verbindung, die Schmelzpunkte ("Smp.") sind in °C angegeben. Wo angegeben, steht A und B für die Diastereomeren.

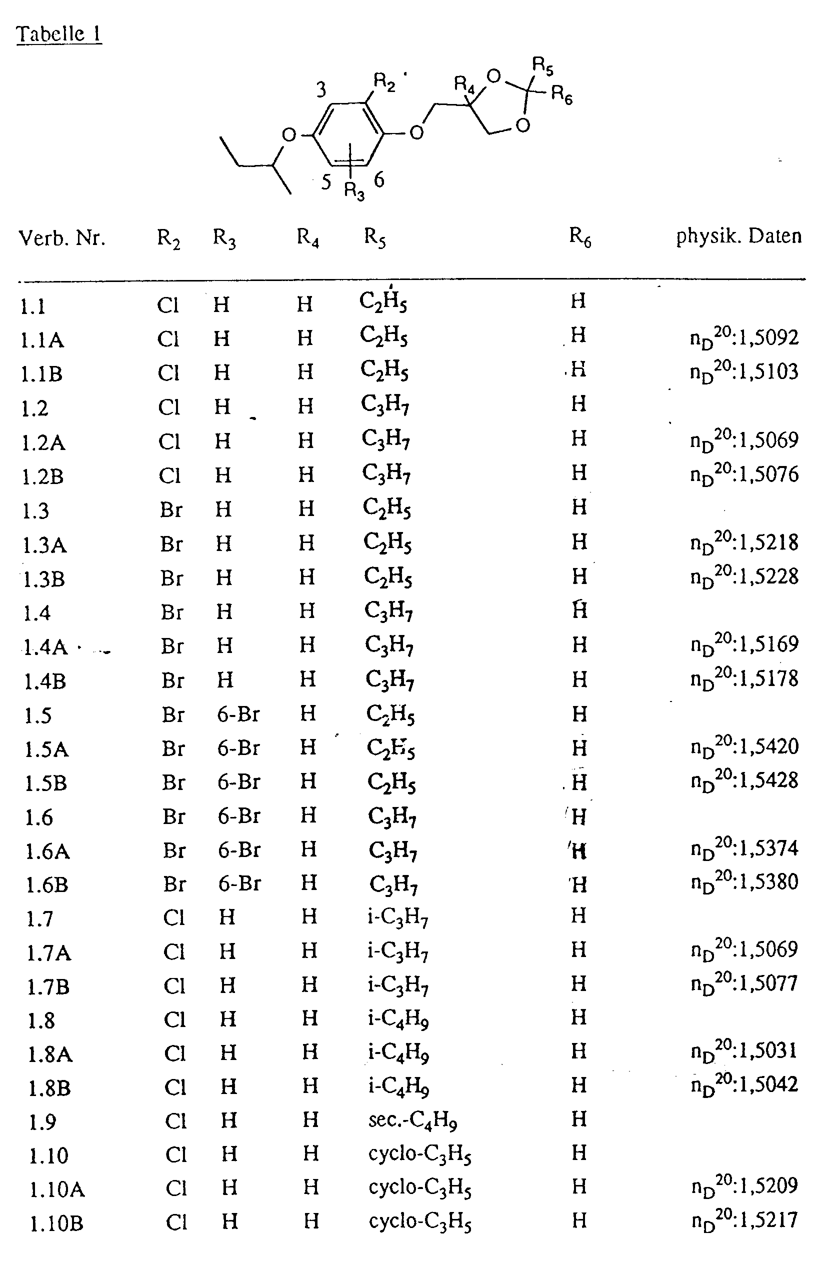

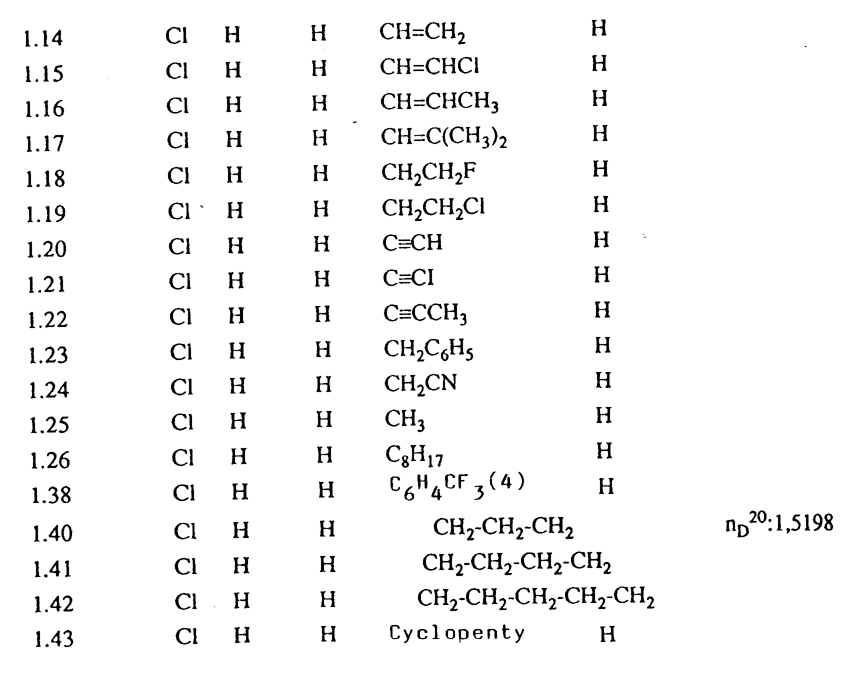

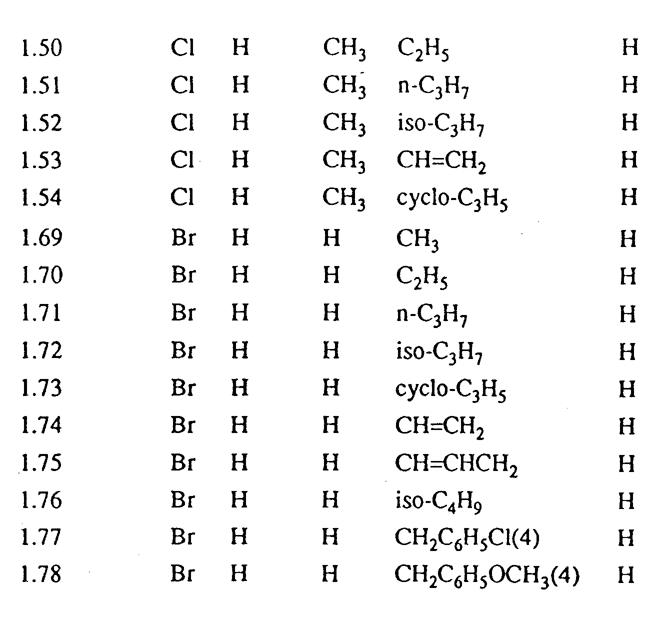

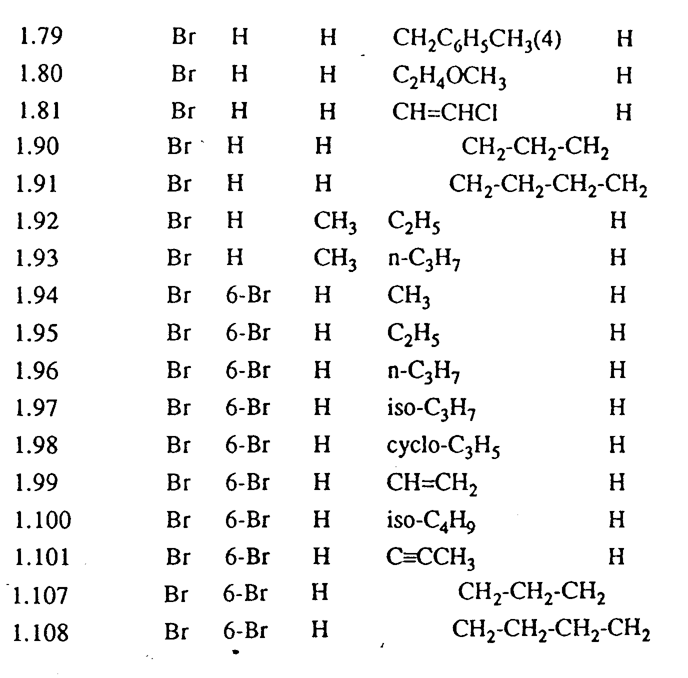

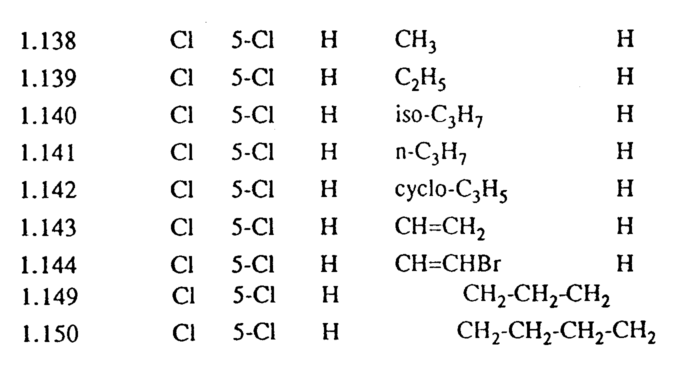

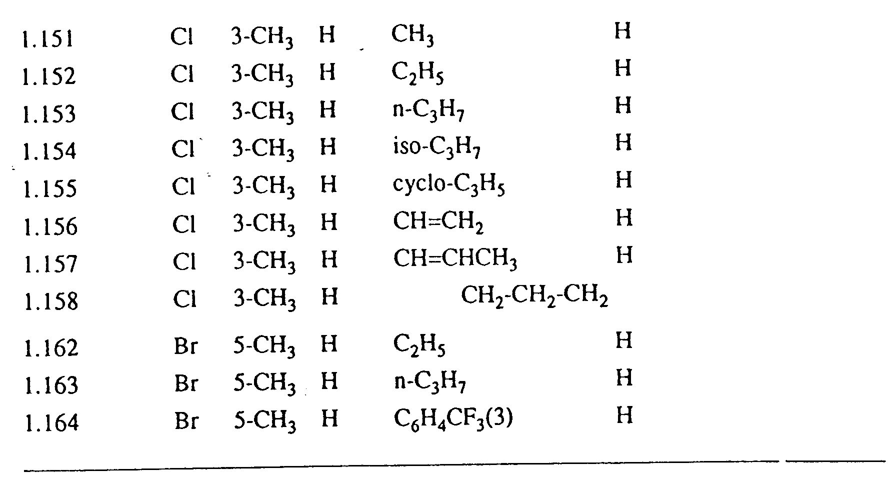

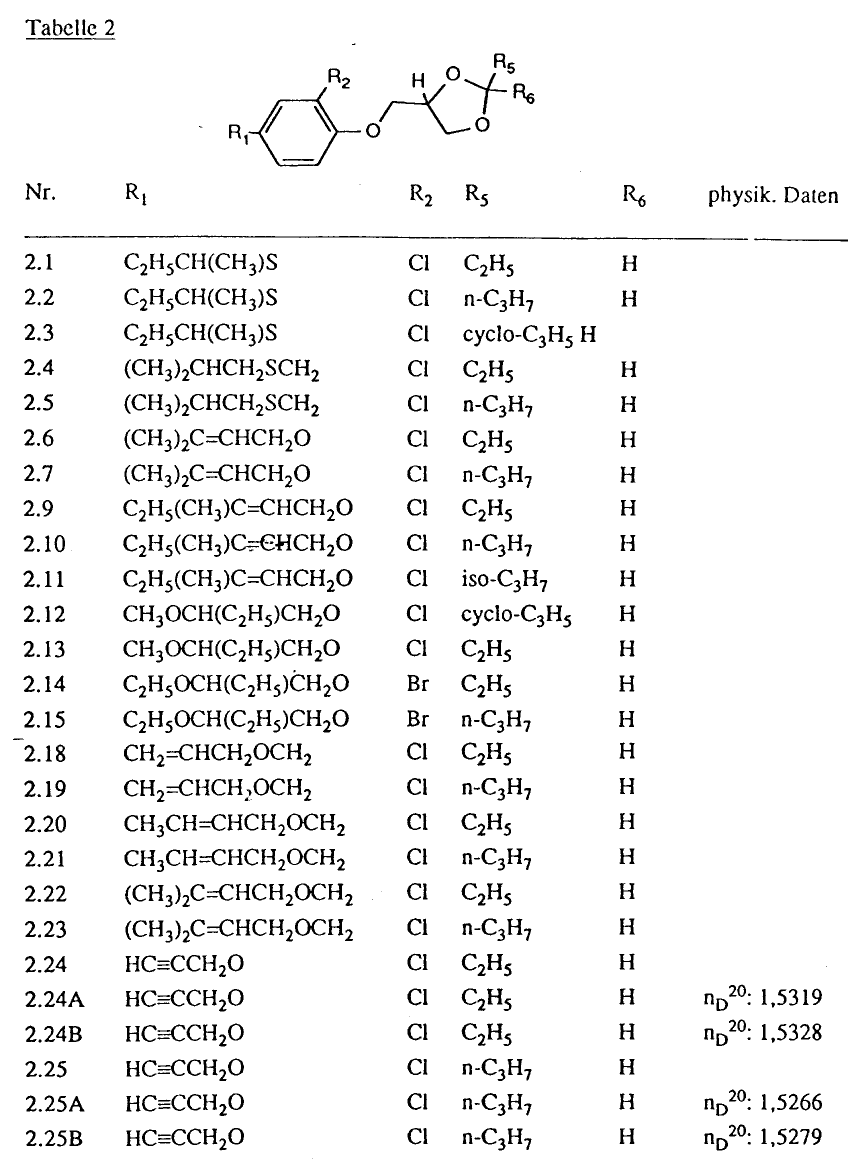

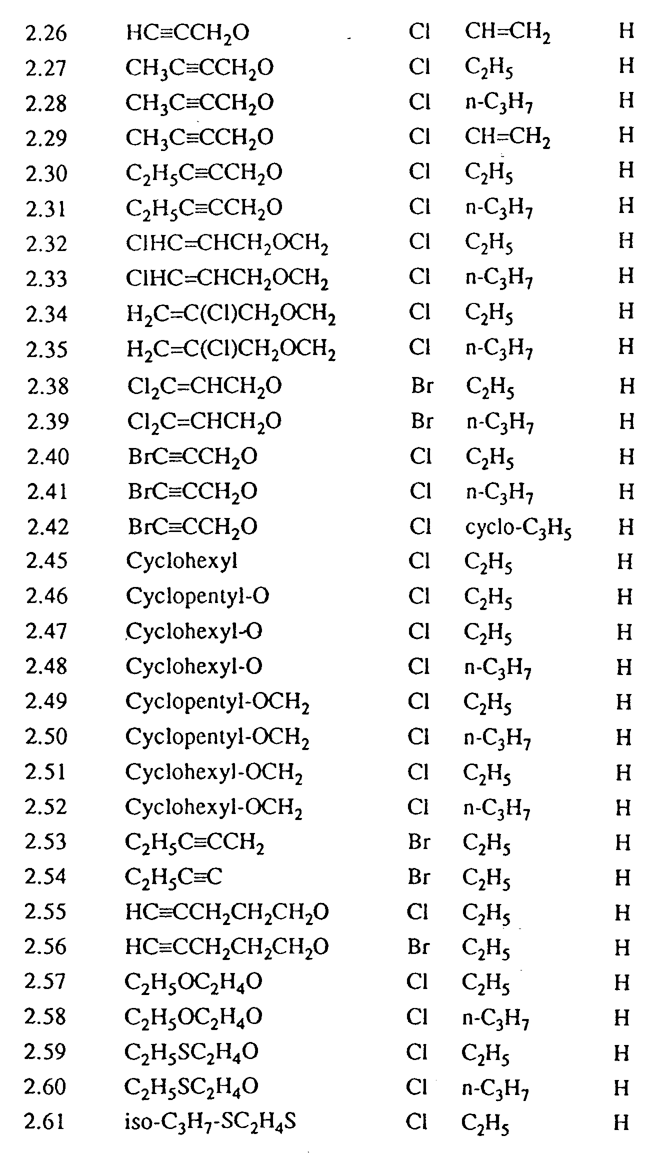

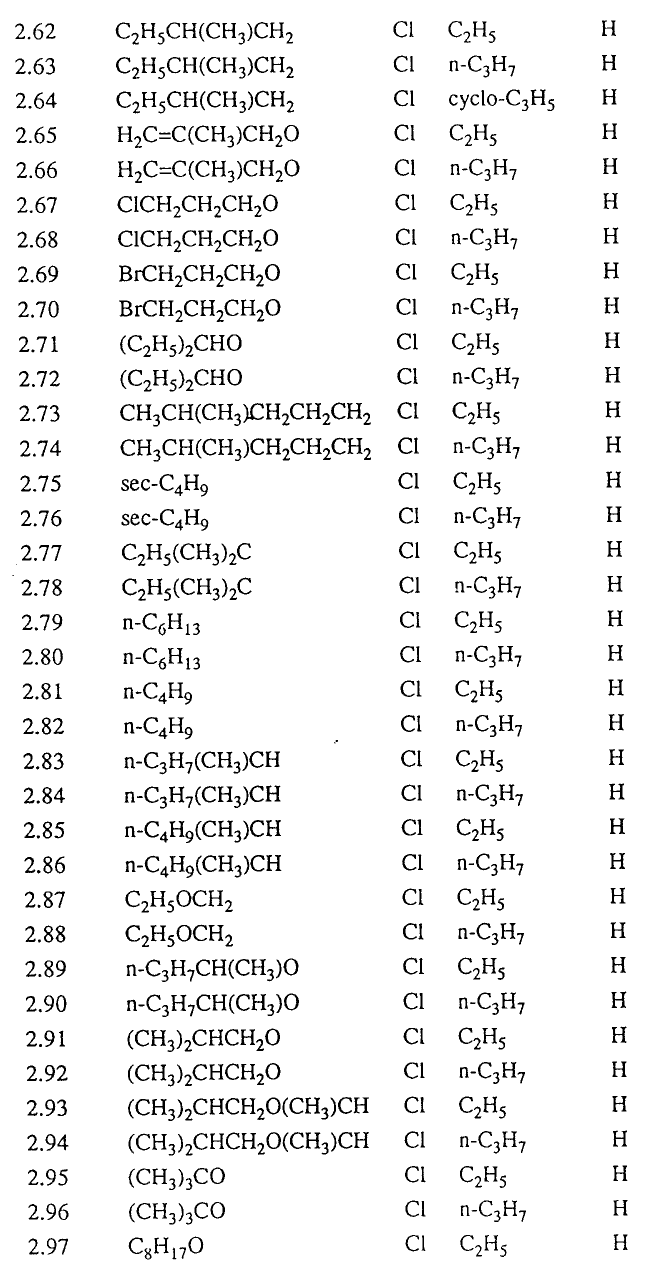

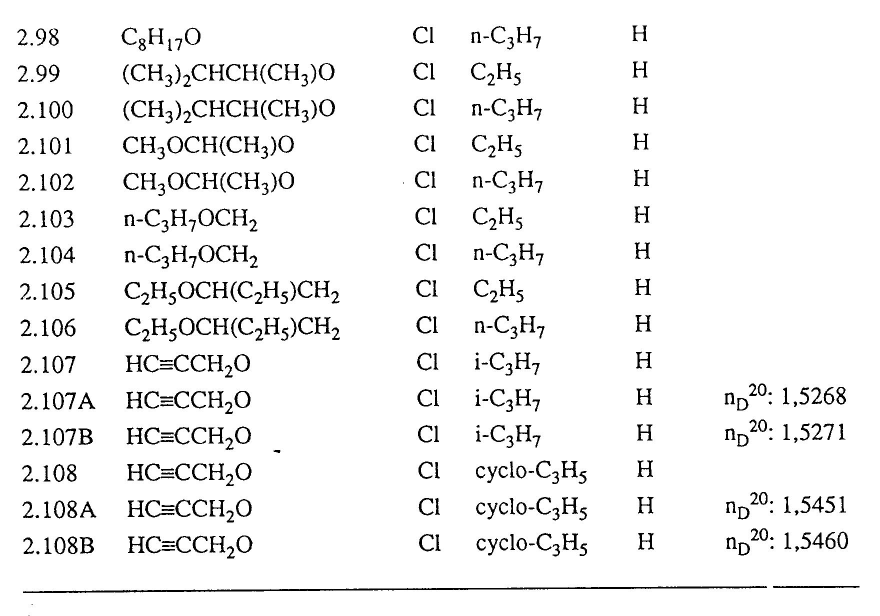

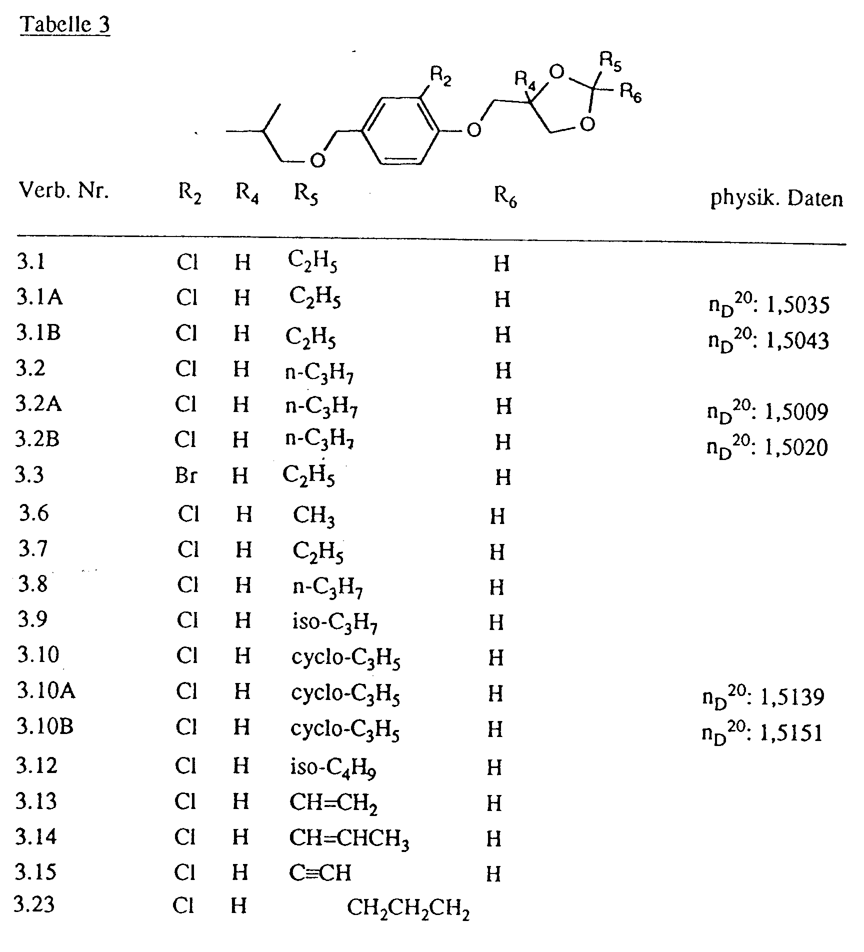

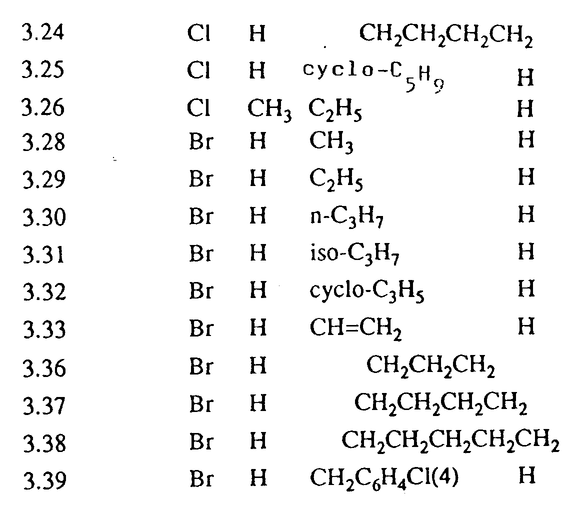

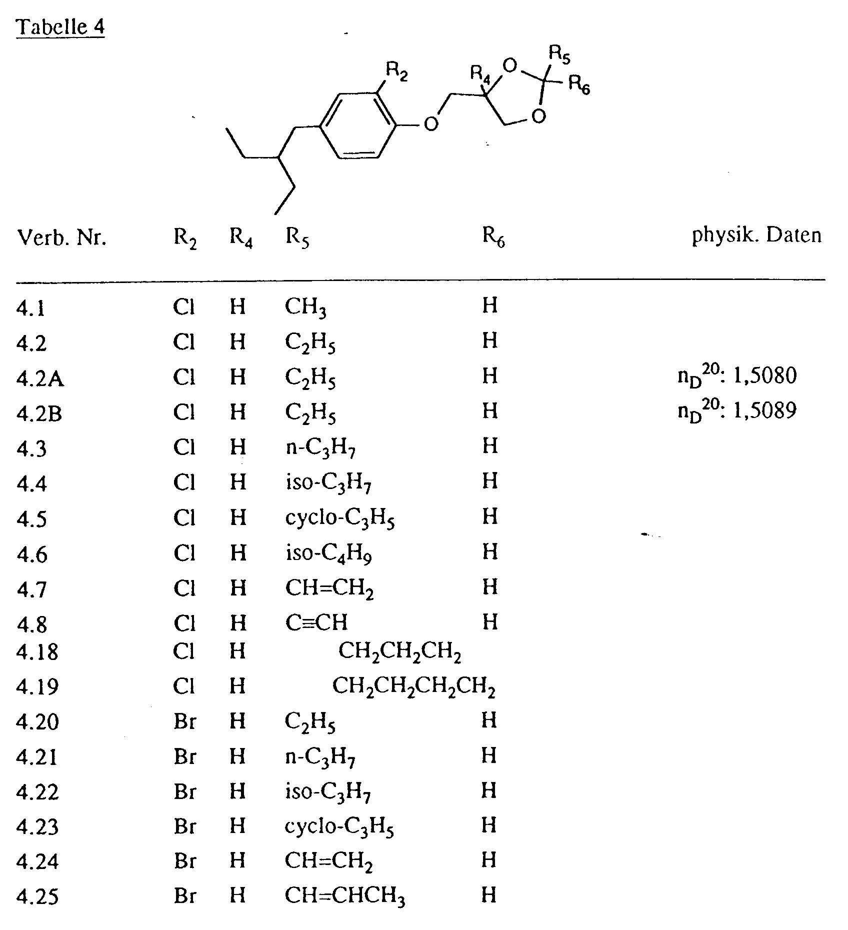

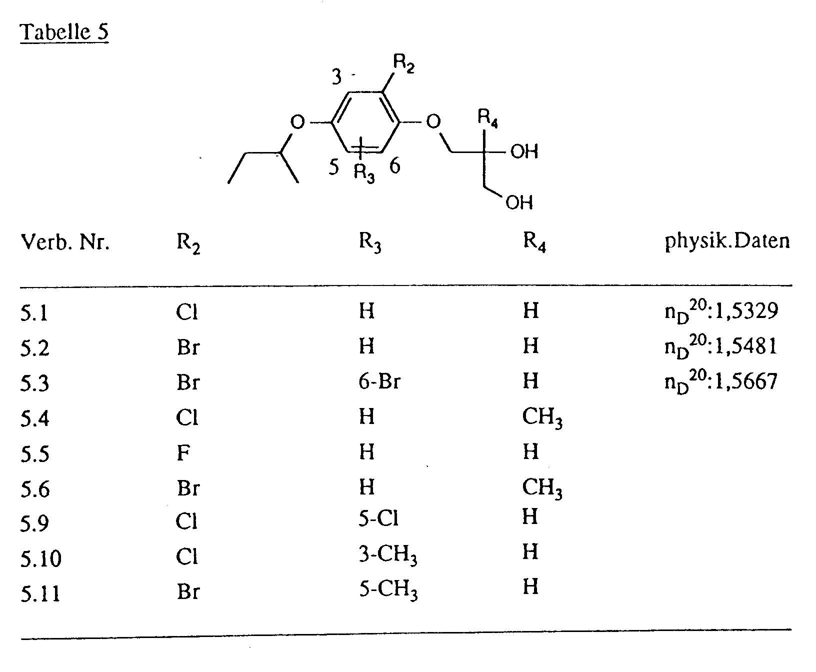

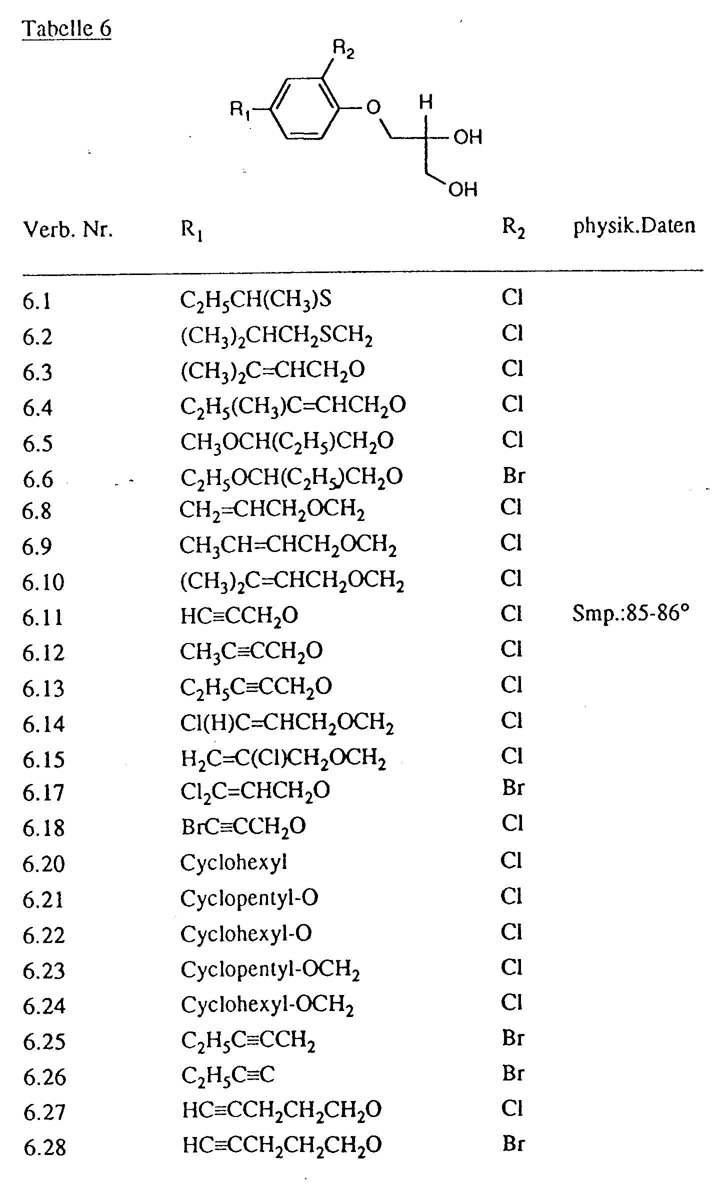

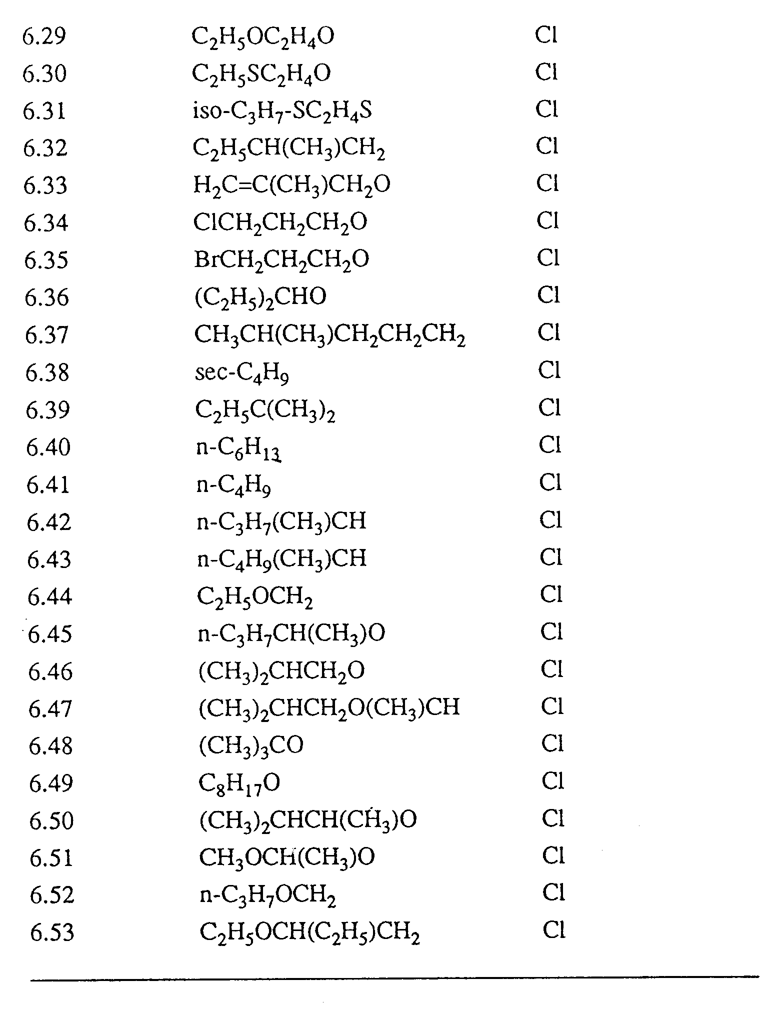

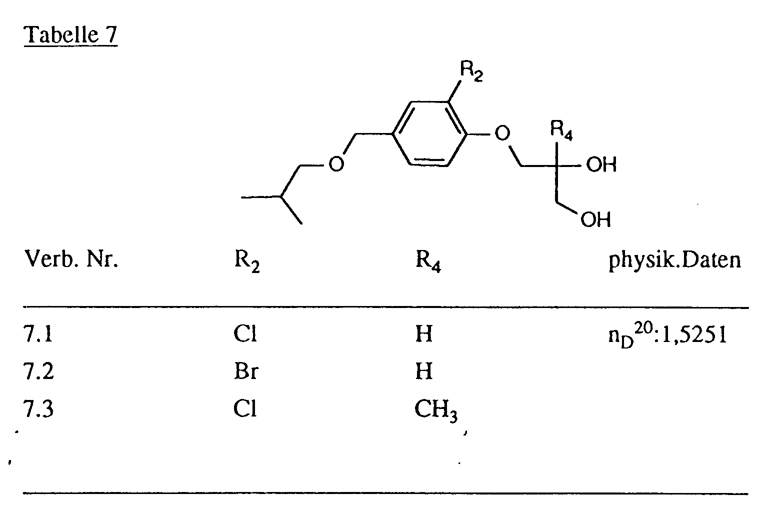

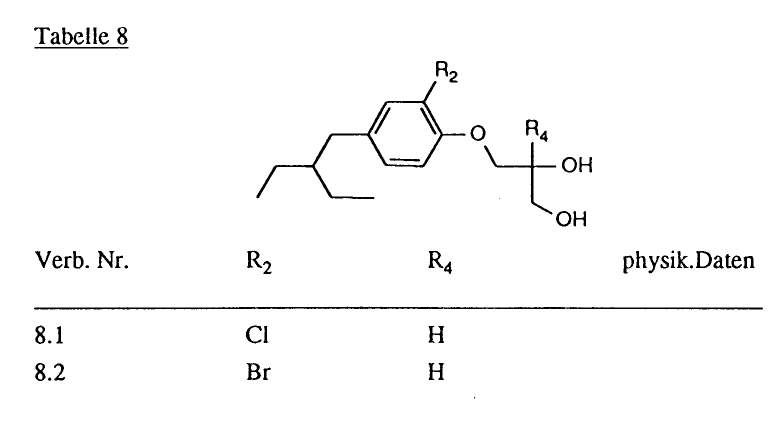

### Formulierungsbeispiele (% = Gewichtsprozent)

| Beispiel F1: Emulsions-Konzentrate | a) | b) | c) |
|---|---|---|---|
| Wirkstoff | 25% | 40% | 50% |
| Calciumdodecylbenzolsulfonat | 5% | 8% | 6% |
| Ricinusölpolyethylenglykolether (36 mol EO) | 5% | - | - |
| Tributylphenolpolyethylenglykolether (30 mol EO) | - | 12% | 4% |
| Cyclohexanon | - | 15% | 20% |
| Xylolgemisch | 65% | 25% | 20% |

Mischen von fein gemahlenem Wirkstoff und Zusatzstoffen ergibt ein Emulsions-Konzentrat, das durch Verdünnen mit Wasser Emulsionen gewünschter Konzentration liefert.

| Beispiel F2: Lösungen | a) | b) | c) | d) |
|---|---|---|---|---|
| Wirkstoff | 80% | 10% | 5% | 95% |
| Ethylenglykolmonomethylether | 20% | - | - | - |
| Polyethylenglykol (MG 400) | - | 70% | - | - |
| N-Methylpyrrolid-2-on | - | 20% | - | - |
| Epoxidiertes Kokosnussöl | - | - | 1% | 5% |
| Benzin (Siedegrenzen: 160-190°) | - | - | 94% | - |

Mischen von fein gemahlenem Wirkstoff und Zusatzstoffen ergibt eine Lösung, die zur Anwendung in Form kleinster Tropfen geeignet ist.

| Beispiel F3: Granulate | a) | b) | c) | d) |
|---|---|---|---|---|
| Wirkstoff | 5% | 10% | 8% | 21% |
| Kaolin | 94% | - | 79% | 54% |
| Hochdisperse Kieselsäure | 1% | - | 13% | 7% |
| Attapulgit | - | 90% | - | 18% |

Der Wirkstoff wird in Dichlormethan gelöst, die Lösung auf das Trägerstoffgemisch aufgesprüht und das Lösungsmittel im Vakuum abgedampft.

| Beispiel F4: Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Mischen von Wirkstoff und Trägerstoffen ergibt gebrauchsfertige Stäubemittel.

| Beispiel F5: Spritzpulver | a) | b) | c) |
|---|---|---|---|
| Wirkstoff | 25% | 50% | 75% |
| Natriumligninsulfonat | 5% | 5% | - |
| Natriumlaurylsulfat | 3% | - | 5% |
| Natriumdiisobutylnaphthalinsulfonat | - | 6% | 10% |
| Octylphenolpolyethylenglykolether (7-8 mol EO) | - | 2% | - |
| Hochdisperse Kieselsäure | 5% | 10% | 10% |
| Kaolin | 62% | 27% | - |

Wirkstoff und Zusatzstoffe werden gemischt und das Gemisch in einer geeigneten Mühle vermahlen. Man erhält Spritzpulver, die sich mit Wasser zu Suspensionen gewünschter Konzentration verdünnen lassen.

| Beispiel F6: Emulsions-Konzentrat | |
|---|---|
| Wirkstoff | 10% |
| Octylphenolpolyethylenglykolether (4-5 mol EO) | 3% |
| Calciumdodecylbenzolsulfonat | 3% |
| Ricinusölpolyethylenglykolether (36 mol EO) | 4% |
| Cyclohexanon | 30% |
| Xylolgemisch | 50% |

Mischen von fein gemahlenem Wirkstoff und Zusatzstoffen ergibt ein Emulsions-Konzentrat, das durch Verdünnen mit Wasser Emulsionen gewünschter Konzentration liefert.

| Beispiel F7: Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem man Wirkstoff und Trägerstoff mischt und das Gemisch in einer geeigneten Mühle vermahlt.

| Beispiel F8: Extruder-Granulat | |
|---|---|
| Wirkstoff | 10% |
| Natriumligninsulfonat | 2% |
| Carboxymethylcellulose | 1% |
| Kaolin | 87% |

Wirkstoff und Zusatzstoffe werden gemischt, das Gemisch vermahlen, mit Wasser angefeuchtet, extrudiert und granuliert und das Granulat im Luftstrom getrocknet.

| Beispiel F9: Umhüllungs-Granulat | |
|---|---|
| Wirkstoff | 3% |
| Polyethylenglykol (MG 200) | 3% |
| Kaolin | 94% |

Gleichmässiges Auftragen des fein gemahlenen Wirkstoffs auf das mit Polyethylenglykol angefeuchtete Kaolin in einem Mischer ergibt staubfreie Umhüllungs-Granulate.

| Beispiel F10: Suspensions-Konzentrat | |
|---|---|
| Wirkstoff | 40% |
| Ethylenglykol | 10% |
| Nonylphenolpolyethylenglykolether (15 mol EO) | 6% |
| Natriumligninsulfonat | 10% |
| Carboxymethylcellulose | 1% |
| wässrige Formaldehydlösung (37%) | 0,2% |
| wässrige Silikonölemulsion (75%) | 0,8% |
| Wasser | 32% |

Mischen von fein gemahlenem Wirkstoff und Zusatzstoffen ergibt ein Suspensions-Konzentrat, das durch Verdünnen mit Wasser Suspensionen gewünschter Konzentration liefert.

### Biologische Beispiele:

### Beispiel B1: Wirkung gegen Boophilus microplus

Vollgesogene adulte Zecken (Weibchen) werden auf eine PVC-Platte geklebt und mit einem Wattebausch überdeckt. Zur Behandlung werden die Testtiere mit 10 ml einer wässrigen Testlösung, die 125 ppm des zu prüfenden Wirkstoffes enthält, übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.
Verbindungen gemäss den Tabellen 1 bis 4 zeigen in diesem Test gute Wirkung. Insbesondere zeigen die Verbindungen Nr. 1.1A, 1.1B, 1.2A und 1.2B eine Wirkung von mehr als 80%.

### Beispiel B2: Ovizide Wirkung auf Cydia pomonella

Auf Filterpapier abgelegte Eier von Cydia pomonella werden für eine kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).
Verbindungen gemäss den Tabellen 1 bis 4 zeigen in diesem Test gute Wirkung. Insbesondere zeigen die Verbindungen Nr. 1.1A, 1.1B, 1.2A und 1.2B eine Wirkung von mehr als 80%.

### Beispiel B3: Ovizide Wirkung auf Adoxophyes reticulana

Auf Filterpapier abgelegte Eier von Adoxophyes reticulana werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).
Verbindungen gemäss den Tabellen 1 bis 4 zeigen in diesem Test gute Wirkung. Insbesondere zeigen die Verbindungen Nr. 1.1A, 1.1B, 1.2A und 1.2B eine Wirkung von mehr als 80%.

### Beispiel B4: Ovizide Wirkung auf Lobesia botrana

Auf Filterpapier abgelegte Eier von Lobesia botrana werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).
Verbindungen gemäss den Tabellen 1 bis 4 zeigen in diesem Test gute Wirkung.

### Beispiel B5: Wirkung gegen Aonidiella aurantii

Kartoffelknollen werden mit Wanderlarven ("Crawlern") von Aonidiella aurantii (rote Citrus-Schildlaus) besiedelt. Nach etwa 2 Wochen werden die Kartoffeln in eine wässrige Emulsions- resp. Suspensions- Spritzbrühe getaucht, die den zu prüfenden Wirkstoff in einer Konzentration von 400 ppm enthält. Nach dem Abtrocknen der so behandelten Kartoffelknollen werden diese in einem Plastikbehälter inkubiert. Zur Auswertung wird 10-12 Wochen später die Ueberlebensrate der Wanderlarven der ersten Folgegeneration der behandelten Schildlaus-Population mit derjenigen der unbehandelten Kontrollansätze verglichen.
Verbindungen gemäss den Tabellen 1 bis 4 zeigen in diesem Test gute Wirkung.

### Beispiel B6: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.
Verbindungen gemäss den Tabellen 1 bis 4 zeigen in diesem Test gute Wirkung. Insbesondere zeigen die Verbindungen Nr. 1.1A, 1.1B, 1.2A und 1.2B eine Wirkung von mehr als 80%.

### Beispiel B7: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.
Verbindungen gemäss den Tabellen 1 bis 4 zeigen in diesem Test gute Wirkung.

### Beispiel B8: Wirkung gegen Bemisia tabaci

Buschbohnen-Pflanzen werden in Gazekäfige gestellt und mit Adulten von Bemisia tabaci (Weisse Fliege) besiedelt. Nach erfolgter Eiablage werden alle Adulten entfernt und 10 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu den unbehandelten Kontrollansätzen.
Verbindungen gemäss den Tabellen 1 bis 4 zeigen in diesem Test gute Wirkung.

### Beispiel B9: Wirkung gegen Tetranychus urticae

Junge Bohnenpflanzen werden mit einer Mischpopulation von Tetranychus urticae besiedelt und 1 Tag später mit einer wässrigen Emulsions- Spritzbrühe, die 400 ppm des Wirkstoffes enthält, besprüht. Die Pflanzen werden anschliessend für 6 Tage bei 25°C inkubiert und danach ausgewertet. Aus dem Vergleich der Anzahl toter Eier, Larven und Adulten auf den behandelten zu denjenigen auf den unbehandelten Pflanzen wird die prozentuale Reduktion der Population (% Wirkung) bestimmt.
Verbindungen gemäss den Tabellen 1 bis 4 zeigen in diesem Test gute Wirkung.

### Beispiel B10: Ovo/larvizide Wirkung auf Heliothis virescens

Auf Baumwolle abgelegte Eier von Heliothis virescens werden mit einer wässrigen Emulsions-Spritzbrühe, die 400 ppm des Wirkstoffes enthält, besprüht. Nach 8 Tagen wird der prozentuale Schlupf der Eier und die Ueberlebensrate der Raupen im Vergleich zu unbehandelten Kontrollansätzen ausgewertet (% Reduktion der Population).
Verbindungen gemäss den Tabellen 1 bis 4 zeigen in diesem Test gute Wirkung.

### Beispiel B11: Wirkung gegen Panonychus ulmi(OP und Carb. resistent)

Apfelsämlinge werden mit adulten Weibchen von Panonychus ulmi besiedelt. Nach sieben Tagen werden die infizierten Pflanzen mit einer wässrigen Emulsion-Spritzbrühe, enthaltend 400 ppm der zu prüfenden Verbindung, bis zur Tropfennässe besprüht und im Gewächshaus kultiviert. Nach 14 Tagen erfolgt die Auswertung. Aus dem Vergleich der Anzahl toter Spinnmilben auf den behandelten zu denjenigen auf den unbehandelten Pflanzen wird die prozentuale Reduktion an Population (% Wirkung) bestimmt.
Verbindungen gemäss den Tabellen 1 bis 4 zeigen in diesem Test gute Wirkung.

### Beispiel B12: Wirkung gegen Ctenocephalides felis

20 bis 25 Floheier werden in eine waagrecht stehend 50-ml-Zellkulturflasche gegeben, in der zuvor 15 g Flohlarven-Nährmedium, welches 100 ppm des zu prüfenden Wirkstoffs enthält, vorgelegt wurde. Die Testflaschen werden in einem Brutschrank bei 26 bis 27°C und 60-70 % Luftfeuchtigkeit bebrütet. Nach 21 Tagen wird die Anwesenheit von adulten Flöhen, nicht geschlüpften Puppen und Larven überprüft.
Verbindungen gemäss den Tabellen 1 bis 4 zeigen in diesem Test gute Wirkung.

### Beispiel B13: Wirkung gegen Bemisia tabaci Eier

Buschbohnen-Pflanzen werden in Gazekäfige gestellt und mit Adulten von Bemisia tabaci (Weisse Fliege) besiedelt. Nach erfolgter Eiablage werden alle Adulten entfernt und 2 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 10 Tage nach der Wirkstoff-Applikation auf %-Schlupf im Vergleich zu den unbehandelten Kontrollansätzen.
Verbindungen gemäss den Tabellen 1 bis 4 zeigen in diesem Test gute Wirkung.

## Patentansprüche

1. Eine Verbindung der Formel 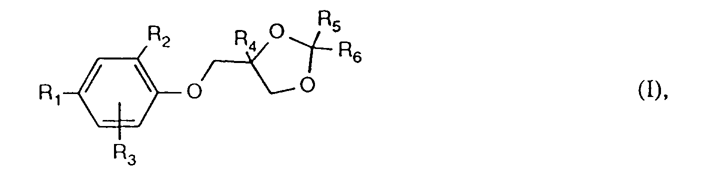 worin
R₁ gegebenenfalls substituiertes C₁-C₈-Alkyl, gegebenenfalls substituiertes C₃-C₈-Cycloalkyl, gegebenenfalls substituiertes C₂-C₈-Alkenyl, gegebenenfalls substituiertes C₂-C₈-Alkinyl, gegebenenfalls substituiertes C₁-C₈-Alkoxy, gegebenenfalls substituiertes C₃-C₈-Cycloalkoxy, gegebenenfalls substituiertes C₂-C₈-Alkenyloxy, gegebenenfalls substituiertes C₂-C₈-Alkinyloxy oder gegebenenfalls substituiertes C₁-C₈-Alkylthio;
R₂ Chlor oder Brom;
R₃ H, Halogen oder Methyl;
R₄ H oder Methyl;
R₅ C₁-C₈-Alkyl, C₃-C₆-Cycloalkyl, C₂-C₅-Alkenyl, C₂-C₅-Alkinyl, C₁-C₃-Alkoxy-C₁-C₃-alkyl, C₁-C₄-Cyanalkyl, C₁-C₆-Halogenalkyl, C₂-C₅-Halogenalkenyl, C₂-C₃-Halogenalkinyl, C₁-C₃-Alkoxycarbonyl, Trifluormethylphenyl, Benzyl oder substituiertes Benzyl, wobei die Substituenten ausgewählt sind aus der Gruppe, bestehend aus C₁-C₃-Alkyl, C₁-C₃-Alkoxy und Halogen; und
R₆ H, Pyridyl, Halogenpyridyl, Furyl, Thienyl oder R₅ und R₆ zusammen geradkettiges C₃-C₅-Alkylen bedeuten.

2. Eine Verbindung gemäss Anspruch 1 der Formel I, worin
R₁ C₁-C₈-Alkyl, C₃-C₈-Cycloalkyl, C₂-C₈-Alkenyl, C₂-C₈-Alkinyl, C₁-C₈-Alkoxy, C₃-C₈-Cycloalkoxy, C₂-C₈-Alkoxy-C₁-C₄-alkyl, C₁-C₈-Alkoxy-C₂-C₄-alkoxy-C₁-C₄-alkyl, C₁-C₈-Alkoxy-C₂-C₄-alkoxy, C₄-C₈-Cycloalkoxy-C₁-C₃-alkyl, C₃-C₈-Alkenyloxy, C₃-C₈-Alkenyloxy-C₁-C₃-alkyl, C₃-C₈-Alkinyloxy, C₃-C₈-Alkinyloxy-C₁-C₃-alkyl, C₃-C₈-Halogenalkyl, C₃-C₈-Halogenalkenyl, C₃-C₈-Halogenalkinyl, C₃-C₈-Halogenalkoxy, C₃-C₈-Halogenalkoxy-C₁-C₃-alkyl, C₃-C₈-Halogenalkenyloxy, C₃-C₈-Halogenalkenyloxy-C₁-C₃-alkyl, C₃-C₈-Halogenalkinyloxy, C₃-C₈-Halogenalkinyloxy-C₁-C₃-alkyl, C₃-C₈-Alkylthio, C₃-C₈-Alkylthio-C₁-C₃- alkyl, C₂-C₈-Alkylthio-C₂-C₃-alkoxy, C₂-C₈-Alkylthio-C₂-C₃-alkoxy-C₁-C₄-alkyl, C₂-C₈-Alkylthio-C₂-C₃-alkylthio, C₂-C₈-Alkylthio-C₂-C₃-alkylthio-C₁-C₄-alkyl, C₂-C₈-Alkoxy-C₂-C₃-alkylthio oder C₂-C₈-Alkoxy-C₂-C₃-alkylthio-C₁-C₄-alkyl;
R₂ Chlor oder Brom;
R₃ H, Halogen oder Methyl;
R₄ H oder Methyl;
R₅ C₁-C₈-Alkyl, C₃-C₆-Cycloalkyl, C₂-C₄-Alkenyl, C₂-C₃-Alkinyl, C₁-C₃-Alkoxy-C₁-C₃-alkyl, C₂-C₄-Cyanalkyl, C₂-C₆-Halogenalkyl, C₂-C₅-Halogenalkenyl, C₂-C₃-Halogenalkinyl, C₁-C₃-Alkoxycarbonyl, Trifluormethylphenyl, Benzyl oder substituiertes Benzyl, wobei die Substituenten aus der Gruppe, bestehend aus C₁-C₃-Alkyl, C₁-C₃-Alkoxy und Chlor ausgewählt sind; und
R₆ H, Pyridyl, Halogenpyridyl, Furyl oder R₅ und R₆ zusammen C₃-C₅-Alkylen bedeuten.

3. Eine Verbindung gemäss Anspruch 1 der Formel I, worin
R₁ C₃-C₈-Alkyl, C₃-C₈-Cycloalkyl, C₃-C₈-Alkenyl, C₃-C₈-Alkinyl, C₃-C₈-Alkoxy, C₃-C₈-Cycloalkoxy, C₂-C₈-Alkoxy-C₁-C₄-alkyl, C₁-C₈-Alkoxy-C₂-C₄-alkoxy-C₁-C₄-alkyl, C₁-C₈-Alkoxy-C₂-C₄-alkoxy, C₄-C₈-Cycloalkoxy-C₁-C₃-alkyl, C₃-C₈-Alkenyloxy, C₃-C₈-Alkenyloxy-C₁-C₃-alkyl, C₃-C₈-Alkinyloxy, C₃-C₈-Alkinyloxy-C₁-C₃-alkyl, C₃-C₈-Halogenalkyl, C₃-C₈-Halogenalkenyl, C₃-C₈-Halogenalkinyl, C₃-C₈-Halogenalkoxy, C₃-C₈-Halogenalkoxy-C₁-C₃-alkyl, C₃-C₈-Halogenalkenyloxy, C₃-C₈-Halogenalkenyloxy-C₁-C₃-alkyl, C₃-C₈-Halogenalkinyloxy, C₃-C₈-Halogenalkinyloxy-C₁-C₃-alkyl, C₃-C₈-Alkylthio, C₃-C₈-Alkylthio-C₁-C₃- alkyl, C₂-C₈-Alkylthio-C₂-C₃-alkoxy, C₂-C₈-Alkylthio-C₂-C₃-alkoxy-C₁-C₄-alkyl, C₂-C₈-Alkylthio-C₂-C₃-alkylthio, C₂-C₈-Alkylthio-C₂-C₃-alkylthio-C₁-C₄-alkyl, C₂-C₈-Alkoxy-C₂-C₃-alkylthio oder C₂-C₈-Alkoxy-C₂-C₃-alkylthio-C₁-C₄-alkyl;
R₂ Chlor oder Brom;
R₃ H, Halogen oder Methyl;
R₄ H oder Methyl;
R₅ C₁-C₈-Alkyl, C₃-C₆-Cycloalkyl, C₂-C₅-Alkenyl, C₂-C₅-Alkinyl, C₁-C₃-Alkoxy-C₁-C₃-alkyl, C₂-C₄-Cyanalkyl, C₂-C₆-Halogenalkyl, C₂-C₅-Halogenalkenyl, C₂-C₃-Halogenalkinyl, C₁-C₃-Alkoxycarbonyl, Trifluormethylphenyl, Benzyl oder substituiertes Benzyl, wobei die Substituenten ausgewählt sind aus der Gruppe, bestehend aus C₁-C₃-Alkyl, C₁-C₃-Alkoxy und Halogen; und
R₆ H, Pyridyl, Halogenpyridyl, Furyl oder R₅ und R₆ zusammen geradkettiges C₃-C₅-Alkylen bedeuten.

4. Eine Verbindung gemäss Anspruch 1 der Formel I, worin
R₁ C₃-C₆-Alkyl, C₃-C₆-Cycloalkyl, C₃-C₆-Alkenyl, C₃-C₆-Alkinyl, C₃-C₈-Alkoxy, C₃-C₆-Cycloalkoxy, C₂-C₆-Alkoxy-C₁-C₄-alkyl, C₁-C₆-Alkoxy-C₂-C₄-alkoxy, C₄-C₆-Cycloalkoxy-C₁-C₃-alkyl, C₃-C₆-Alkenyloxy, C₃-C₆-Alkenyloxy-C₁-C₃-alkyl, C₃-C₆-Alkinyloxy, C₃-C₆-Alkinyloxy-C₁-C₃-alkyl, C₃-C₆-Halogenalkyl, C₃-C₆-Halogenalkenyl, C₃-C₆-Halogenalkinyl, C₃-C₆-Halogenalkoxy, C₃-C₆-Halogenalkoxy-C₁-C₃-alkyl, C₃-C₆-Halogenalkenyloxy, C₃-C₆-Halogenalkenyloxy-C₁-C₃-alkyl, C₃-C₆-Halogenalkinyloxy, C₃-C₆-Halogenalkinyloxy-C₁-C₃-alkyl, C₃-C₆-Alkylthio, C₃-C₆-Alkylthio-C₁-C₃-alkyl, C₂-C₆-Alkylthio-C₂-C₃-alkoxy oder C₂-C₆-Alkylthio-C₂-C₃-alkylthio;
R₂ Chlor oder Brom;
R₃ H, Halogen oder Methyl;
R₄ H oder Methyl;
R₅ C₁-C₈-Alkyl, C₃-C₆-Cycloalkyl, C₂-C₄-Alkenyl, C₂-C₃-Alkinyl, C₁-C₃-Alkoxy-C₁-C₃-alkyl, C₂-C₄-Cyanalkyl, C₂-C₆-Halogenalkyl, C₂-C₅-Halogenalkenyl, C₂-C₃-Halogenalkinyl, C₁-C₃-Alkoxycarbonyl, Trifluormethylphenyl, Benzyl oder substituiertes Benzyl, wobei die Substituenten aus der Gruppe, bestehend aus C₁-C₃-Alkyl, C₁-C₃-Alkoxy und Chlor ausgewählt sind; und
R₆ H, Pyridyl, Halogenpyridyl, Furyl oder R₅ und R₆ zusammen C₃-C₅-Alkylen bedeuten.

5. Eine Verbindung gemäss Anspruch 1 der Formel I, worin
R₁ C₄-C₆-Alkyl, C₄-C₆-Cycloalkyl, C₃-C₆-Alkenyl, C₃-C₅-Alkinyl, C₄-C₈-Alkoxy, C₅-C₆-Cycloalkoxy, C₂-C₄-Alkoxy-C₁-C₄-alkyl, C₁-C₃-Alkoxy-C₂-C₄-alkoxy, C₅-C₆-Cycloalkoxy-C₁-C₂-alkyl, C₄-C₆-Alkenyloxy, C₃-C₅-Alkenyloxy-C₁-C₂-alkyl, C₃-C₅-Alkinyloxy, C₃-C₅-Alkinyloxy-C₁-C₂-alkyl, C₃-C₅-Halogenalkyl, C₃-C₅-Halogenalkenyl, C₃-C₅-Halogenalkinyl, C₃-C₅-Halogenalkoxy, C₃-C₅-Halogenalkoxy-C₁-C₂-alkyl, C₃-C₄-Halogenalkenyloxy, C₃-C₄-Halogenalkenyloxy-C₁-C₂-alkyl, C₃-C₄-Halogenalkinyloxy, C₃-C₄-Halogenalkinyloxy-C₁-C₂-alkyl, C₃-C₅-Alkylthio, C₃-C₅-Alkylthio-C₁-C₂-alkyl, C₂-C₄-Alkylthio-C₂-C₃-alkoxy oder C₂-C₄-Alkylthio-C₂-C₃-alkylthio;
R₂ Chlor oder Brom;
R₃ H, Halogen oder Methyl;
R₄ H oder Methyl;
R₅ C₁-C₈-Alkyl, C₃-C₆-Cycloalkyl, C₂-C₄-Alkenyl, C₂-C₃-Alkinyl, Methoxyethyl, Cyanmethyl, Halogenethyl, Halogenvinyl, Halogenacetylenyl, Trifluormethylphenyl, Benzyl oder substituiertes Benzyl, wobei die Substituenten aus der Gruppe, bestehend aus Methyl, Methoxy und Chlor ausgewählt sind; und
R₆ H, Pyridyl, Halogenpyridyl, Furyl oder R₅ und R₆ zusammen C₃-C₅-Alkylen bedeuten.

6. Eine Verbindung gemäss Anspruch 1 der Formel I, worin
R₁ C₄-C₆-Alkyl, Cyclohexyl, C₄-C₅-Alkinyl, C₄-C₅-Alkoxy, C₅-C₆-Cycloalkoxy, C₂-C₄-Alkoxy-C₁-C₄-alkyl, C₁-C₂-Alkoxyethoxy, C₅-C₆-Cycloalkoxymethyl, C₄-C₆-Alkenyloxy, C₃-C₅-Alkenyloxymethyl, C₃-C₅-Alkinyloxy, C₃-C₄-Halogenalkoxy, C₃-Halogenalkenyloxy, C₃-Halogenalkenyloxymethyl, C₃-Halogenalkinyloxy, C₃-Halogenalkinyloxymethyl, C₄-Alkylthio, C₄-Alkylthiomethyl, C₂-C₃-Alkylthioethoxy oder C₂-C₃-Alkylthioethylthio;
R₂ Chlor oder Brom;
R₃ H, Halogen oder Methyl;
R₄ H oder Methyl;
R₅ C₁-C₈-Alkyl, C₃-C₆-Cycloalkyl, C₂-C₄-Alkenyl, C₂-C₃-Alkinyl, Methoxyethyl, Cyanmethyl, Halogenethyl, Halogenvinyl, Halogenacetylenyl, Trifluormethylphenyl, Benzyl oder substituiertes Benzyl, wobei die Substituenten aus der Gruppe, bestehend aus Methyl, Methoxy und Chlor ausgewählt sind; und
R₆ H, Pyridyl, Halogenpyridyl, Furyl oder R₅ und R₆ zusammen C₃-C₅-Alkylen bedeuten.

7. Eine Verbindung gemäss Anspruch 1 der Formel I, worin
R₁ C₄-C₆-Alkyl, Cyclohexyl, C₄-C₅-Alkinyl, C₄-C₅-Alkoxy, C₅-C₆-Cycloalkoxy, C₂-C₄-Alkoxy-C₁-C₄-alkyl, C₁-C₂-Alkoxyethoxy, C₅-C₆-Cycloalkoxymethyl, C₄-C₆-Alkenyloxy, C₃-C₅-Alkenyloxymethyl, C₃-C₅-Alkinyloxy, C₃-C₄-Halogenalkoxy, C₃-Halogenalkenyloxy, C₃-Halogenalkenyloxymethyl, C₃-Halogenalkinyloxy, C₃-Halogenalkinyloxymethyl, C₄-Alkylthio, C₄-Alkylthiomethyl, C₂-C₃-Alkylthioethoxy oder C₂-C₃-Alkylthioethylthio;
R₂ Chlor oder Brom;
R₃ H, Halogen oder Methyl;
R₄ H oder Methyl;
R₅ C₁-C₈-Alkyl, Cyclopropyl, C₂-C₄-Alkenyl, C₂-C₃-Alkinyl, Methoxyethyl, Cyanmethyl, Fluorethyl, Chlorethyl, Fluorvinyl, Chlorvinyl, Bromvinyl, Iodacetylenyl, Trifluormethylphenyl, Benzyl, Tolyl, Anisyl oder Chlorphenyl; und
R₆ H, Pyridyl, Halogenpyridyl, Furyl oder R₅ und R₆ zusammen C₃-C₅-Alkylen bedeuten.

8. Eine Verbindung gemäss Anspruch 1 der Formel I, worin
R₁ C₄-C₆-Alkyl, Cyclohexyl, C₄-C₅-Alkinyl, C₄-C₅-Alkoxy, C₅-C₆-Cycloalkoxy, C₂-C₄-Alkoxy-C₁-C₄-alkyl, C₁-C₂-Alkoxyethoxy, C₅-C₆-Cycloalkoxymethyl, C₄-C₆-Alkenyloxy, C₃-C₅-Alkenyloxymethyl, C₃-C₅-Alkinyloxy, C₃-C₄-Halogenalkoxy, C₃-Halogenalkenyloxy, C₃-Halogenalkenyloxymethyl, C₃-Halogenalkinyloxy, C₃-Halogenalkinyloxymethyl, C₄-Alkylthio, C₄-Alkylthiomethyl, C₂-C₃-Alkylthioethoxy oder C₂-C₃-Alkylthioethylthio;
R₂ Chlor oder Brom;
R₃ H, Halogen oder Methyl;
R₄ H oder Methyl;
R₅ C₁-C₈-Alkyl, Cyclopropyl, C₂-C₄-Alkenyl, C₂-C₃-Alkinyl, Methoxyethyl, Cyanmethyl, Fluorethyl, Chlorethyl, Fluorvinyl, Chlorvinyl, Bromvinyl, Iodacetylenyl, Trifluormethylphenyl, Benzyl, Tolyl, Anisyl oder Chlorphenyl; und
R₆ H, Pyridyl, Chlorpyridyl, Furyl oder R₅ und R₆ zusammen C₃-C₅-Alkylen bedeuten.

9. Eine Verbindung gemäss Anspruch 1 der Formel I, worin
R₁ sec-Butoxy oder iso-Butoxy;
R₂ Chlor oder Brom;
R₃ H oder Brom;
R₄ und R₆ H; und
R₅ Ethyl oder Propyl bedeuten.

10. Verfahren zur Herstellung einer Verbindung gemäss Anspruch 1 der Formel I, dadurch gekennzeichnet, dass man
a) eine Verbindung der Formel 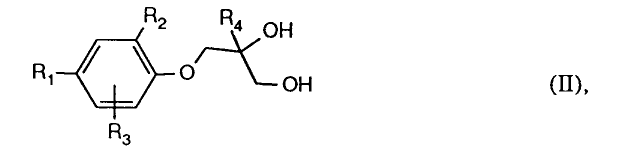 die bekannt ist oder in Analogie zu entsprechenden bekannten Verbindungen hergestellt werden kann und worin R₁, R₂, R₃ und R₄ die für die Formel I angegebenen Bedeutungen haben, in freier Form oder in Salzform, eventuell in Gegenwart eines Säurekatalysators oder eines wasserbindenden Mittels, mit einer Verbindung der Formel
R₅COR₆ (III),
die bekannt ist oder in Analogie zu entsprechenden bekannten Verbindungen hergestellt werden kann und worin R₅ und R₆ die für die Formel I angegebenen Bedeutungen haben, oder mit einer Verbindung der Formel 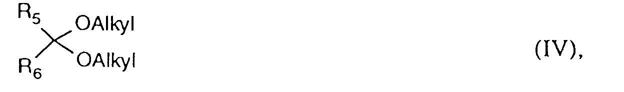 die bekannt ist oder in Analogie zu entsprechenden bekannten Verbindungen hergestellt werden kann und worin R₅ und R₆ die für die Formel I angegebenen Bedeutungen haben und Alkyl Methyl oder Ethyl ist, oder
b) eine Verbindung der Formel 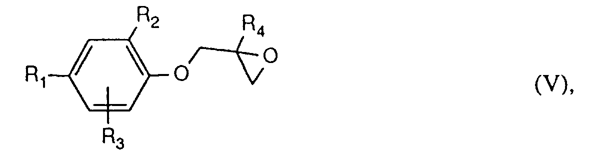 die bekannt ist oder in Analogie zu entsprechenden bekannten Verbindungen hergestellt werden kann und worin R₁, R₂, R₃ und R₄ die für die Formel I angegebenen Bedeutungen haben, eventuell in Gegenwart eines Säurekatalysators oder eines wasserbindenden Mittels, mit einer Verbindung der Formel III, oder
c) eine Verbindung der Formel 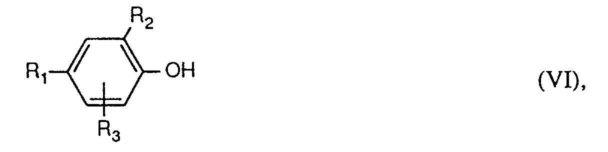 die bekannt ist oder in Analogie zu entsprechenden bekannten Verbindungen hergestellt werden kann und worin R₁, R₂ und R₃ die für die Formel I angegebenen Bedeutungen haben, eventuell in Gegenwart einer Base oder eines wasserbindenden Mittels, mit einer Verbindung der Formel 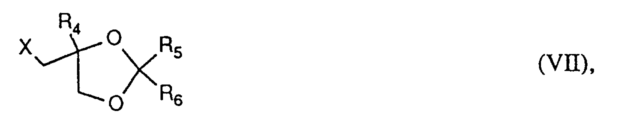 die bekannt ist oder in Analogie zu entsprechenden bekannten Verbindungen hergestellt werden kann und worin R₄, R₅ und R₆ die für die Formel I angegebenen Bedeutungen haben und X eine Abgangsgruppe ist, umsetzt
und/oder, wenn erwünscht, eine verfahrensgemäss oder auf andere Weise erhältliche Verbindung der Formel I in eine andere Verbindung der Formel I überführt und/oder ein verfahrensgemäss erhältliches Gemisch von Isomeren auftrennt und das gewünschte Isomere isoliert.

11. Schädlingsbekämpfungsmittel, dadurch gekennzeichnet, dass es mindestens eine Verbindung gemäss Anspruch 1 der Formel I als Wirkstoff und gegebenenfalls mindestens einen Hilfsstoff enthält.

12. Verfahren zur Herstellung eines mindestens einen Hilfsstoff enthaltenden Mittels gemäss Anspruch 11, dadurch gekennzeichnet, dass man den Wirkstoff mit dem (den) Hilfsstoff(en) innig vermischt und/oder vermahlt.

13. Verwendung einer Verbindung gemäss Anspruch 1 der Formel I zur Herstellung eines Mittels gemäss Anspruch 11.

14. Verwendung eines Mittels gemäss Anspruch 11 zur Bekämpfung von Schädlingen.

15. Verwendung gemäss Anspruch 14 zum Schutz von pflanzlichem Vermehrungsgut.

16. Verfahren zur Bekämpfung von Schädlingen, dadurch gekennzeichnet, dass man ein Mittel gemäss Anspruch 11 auf die Schädlinge oder ihren Lebensraum appliziert.

17. Verfahren gemäss Anspruch 16 zum Schutz von pflanzlichem Vermehrungsgut, dadurch gekennzeichnet, dass man das Vermehrungsgut oder den Ort der Ausbringung des Vermehrungsguts behandelt.

18. Pflanzliches Vermehrungsgut, behandelt gemäss dem in Anspruch 17 beschriebenen Verfahren.

## Claims

1. A compound of the formula 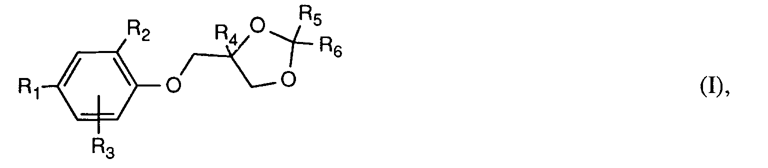 in which
R₁ is substituted or unsubstituted C₁-C₈alkyl, substituted or unsubstituted C₃-C₈cycloalkyl, substituted or unsubstituted C₂-C₈alkenyl, substituted or unsubstituted C₂-C₈alkynyl, substituted or unsubstituted C₁-C₈alkoxy, substituted or unsubstituted C₃-C₈cycloalkoxy, substituted or unsubstituted C₂-C₈alkenyloxy, substituted or unsubstituted C₂-C₈alkynyloxy or substituted or unsubstituted C₁-C₈alkylthio;
R₂ is chlorine or bromine;
R₃ is H, halogen or methyl;
R₄ is H or methyl;
R₅ is C₁-C₈alkyl, C₃-C₆cycloalkyl, C₂-C₅alkenyl, C₂-C₅alkynyl, C₁-C₃alkoxy-C₁-C₃alkyl, C₁-C₄cyanoalkyl, C₁-C₆haloalkyl, C₂-C₅haloalkenyl, C₂-C₃haloalkynyl, C₁-C₃alkoxycarbonyl, trifluoromethylphenyl, benzyl or substituted benzyl, the substituents being selected from the group consisting of C₁-C₃alkyl, C₁-C₃alkoxy and halogen; and
R₆ is H, pyridyl, halopyridyl, furyl, thienyl, or R₅ and R₆ together are straight-chain C₃-C₅alkylene.

2. A compound according to claim 1, of the formula I in which
R₁ is C₁-C₈alkyl, C₃-C₈cycloalkyl, C₂-C₈alkenyl, C₂-C₈alkynyl, C₁-C₈alkoxy, C₃-C₈cycloalkoxy, C₂-C₈alkoxy-C₁-C₄alkyl, C₁-C₈alkoxy-C₂-C₄alkoxy-C₁-C₄alkyl, C₁-C₈alkoxy-C₂-C₄alkoxy, C₄-C₈cycloalkoxy-C₁-C₃alkyl, C₃-C₈alkenyloxy, C₃-C₈alkenyloxy-C₁-C₃alkyl, C₃-C₈alkynyloxy, C₃-C₈alkynyloxy-C₁-C₃alkyl, C₃-C₈haloalkyl, C₃-C₈haloalkenyl, C₃-C₈haloalkynyl, C₃-C₈haloalkoxy, C₃-C₈haloalkoxy-C₁-C₃alkyl, C₃-C₈haloalkenyloxy, C₃-C₈haloalkenyloxyC₁-C₃alkyl, C₃-C₈haloalkynyloxy, C₃-C₈haloalkynyloxy-C₁-C₃alkyl, C₃-C₈alkylthio, C₃-C₈alkylthio-C₁-C₃alkyl, C₂-C₈alkylthio-C₂-C₃alkoxy, C₂-C₈alkylthio-C₂-C₃alkoxy-C₁-C₄alkyl, C₂-C₈alkylthio-C₂-C₃alkylthio, C₂-C₈alkylthio-C₂-C₃alkylthio-C₁-C₄alkyl, C₂-C₈alkoxy-C₂-C₃alkylthio or C₂-C₈alkoxy-C₂-C₃alkylthio-C₁-C₄alkyl;
R₂ is chlorine or bromine;
R₃ is H, halogen or methyl;
R₄ is H or methyl;
R₅ is C₁-C₈alkyl, C₃-C₆cycloalkyl, C₂-C₄alkenyl, C₂-C₃alkynyl, C₁-C₃alkoxy-C₁-C₃alkyl, C₂-C₄cyanoalkyl, C₂-C₆haloalkyl, C₂-C₅haloalkenyl, C₂-C₃haloalkynyl, C₁-C₃alkoxycarbonyl, trifluoromethylphenyl, benzyl or substituted benzyl, the substituents being selected from the group consisting of C₁-C₃alkyl, C₁-C₃alkoxy and chlorine; and
R₆ is H, pyridyl, halopyridyl, furyl or R₅ and R₆ together are C₃-C₅alkylene.

3. A compound according to claim 1, of the formula I in which
R₁ is C₃-C₈alkyl, C₃-C₈cycloalkyl, C₃-C₈alkenyl, C₃-C₈alkynyl, C₃-C₈alkoxy, C₃-C₈cycloalkoxy, C₂-C₈alkoxy-C₁-C₄alkyl, C₁-C₈alkoxy-C₂-C₄alkoxy-C₁-C₄alkyl, C₁-C₈alkoxy-C₂-C₄alkoxy, C₄-C₈cycloalkoxy-C₁-C₃alkyl, C₃-C₈alkenyloxy, C₃-C₈alkenyloxy-C₁-C₃alkyl, C₃-C₈alkynyloxy, C₃-C₈alkynyloxy-C₁-C₃alkyl, C₃-C₈haloalkyl, C₃-C₈haloalkenyl, C₃-C₈haloalkynyl, C₃-C₈haloalkoxy, C₃-C₈haloalkoxy-C₁-C₃alkyl, C₃-C₈haloalkenyloxy, C₃-C₈haloalkenyloxy-C₁-C₃alkyl, C₃-C₈haloalkynyloxy, C₃-C₈haloalkynyloxy-C₁-C₃alkyl, C₃-C₈alkylthio, C₃-C₈alkylthio-C₁-C₃alkyl, C₂-C₈alkylthio-C₂-C₃alkoxy, C₂-C₈alkylthio-C₂-C₃alkoxy-C₁-C₄alkyl, C₂-C₈alkylthio-C₂-C₃alkylthio, C₂-C₈alkylthio-C₂-C₃alkylthio-C₁-C₄alkyl, C₂-C₈alkoxy-C₂-C₃alkylthio or C₂-C₈alkoxy-C₂-C₃alkylthio-C₁-C₄alkyl;
R₂ is chlorine or bromine;
R₃ is H, halogen or methyl;
R₄ is H or methyl;
R₅ is C₁-C₈alkyl, C₃-C₆cycloalkyl, C₂-C₅alkenyl, C₂-C₅alkynyl, C₁-C₃alkoxy-C₁-C₃alkyl, C₂-C₄cyanoalkyl, C₂-C₆haloalkyl, C₂-C₅haloalkenyl, C₂-C₃haloalkynyl, C₁-C₃alkoxycarbonyl, trifluoromethylphenyl, benzyl or substituted benzyl, the substituents being selected from the group consisting of C₁-C₃alkyl, C₁-C₃alkoxy and halogen; and
R₆ is H, pyridyl, halopyridyl, furyl or R₅ and R₆ together are straight-chain C₃-C₅alkylene.

4. A compound according to claim 1, of the formula I in which
R₁ is C₃-C₆alkyl, C₃-C₆cycloalkyl, C₃-C₆alkenyl, C₃-C₆alkynyl, C₃-C₈alkoxy, C₃-C₆cycloalkoxy, C₂-C₆alkoxy-C₁-C₄alkyl, C₁-C₆alkoxy-C₂-C₄alkoxy, C₄-C₆cycloalkoxy-C₁-C₃alkyl, C₃-C₆alkenyloxy, C₃-C₆alkenyloxy-C₁-C₃alkyl, C₃-C₆alkynyloxy, C₃-C₆alkynyloxy-C₁-C₃alkyl, C₃-C₆haloalkyl, C₃-C₆haloalkenyl, C₃-C₆haloalkynyl, C₃-C₆haloalkoxy, C₃-C₆haloalkoxy-C₁-C₃alkyl, C₃-C₆haloalkenyloxy, C₃-C₆haloalkenyloxy-C₁-C₃alkyl, C₃-C₆haloalkynyloxy, C₃-C₆haloalkynyloxy-C₁-C₃alkyl, C₃-C₆alkylthio, C₃-C₆alkylthio-C₁-C₃alkyl, C₂-C₆alkylthio-C₂-C₃alkoxy or C₂-C₆alkylthio-C₂-C₃alkylthio;
R₂ is chlorine or bromine;
R₃ is H, halogen or methyl;
R₄ is H or methyl;
R₅ is C₁-C₈alkyl, C₃-C₆cycloalkyl, C₂-C₄alkenyl, C₂-C₃alkynyl, C₁-C₃alkoxy-C₁-C₃alkyl, C₂-C₄cyanoalkyl, C₂-C₆haloalkyl, C₂-C₅haloalkenyl, C₂-C₃haloalkynyl, C₁-C₃alkoxycarbonyl, trifluoromethylphenyl, benzyl or substituted benzyl, the substituents being selected from the group consisting of C₁-C₃alkyl, C₁-C₃alkoxy and chlorine; and
R₆ is H, pyridyl, halopyridyl, furyl, or R₅ and R₆ together are C₃-C₅alkylene.

5. A compound according to claim 1, of the formula I in which
R₁ is C₄-C₆alkyl, C₄-C₆cycloalkyl, C₃-C₆alkenyl, C₃-C₅alkynyl, C₄-C₈alkoxy, C₅-C₆cycloalkoxy, C₂-C₄alkoxy-C₁-C₄alkyl, C₁-C₃alkoxy-C₂-C₄alkoxy, C₅-C₆cycloalkoxy-C₁-C₂alkyl, C₄-C₆alkenyloxy, C₃-C₅alkenyloxy-C₁-C₂alkyl, C₃-C₅alkynyloxy, C₃-C₅alkynyloxy-C₁-C₂alkyl, C₃-C₅haloalkyl, C₃-C₅haloalkenyl, C₃-C₅haloalkynyl, C₃-C₅haloalkoxy, C₃-C₅haloalkoxy-C₁-C₂alkyl, C₃-C₄haloalkenyloxy, C₃-C₄haloalkenyloxy-C₁-C₂alkyl, C₃-C₄haloalkynyloxy, C₃-C₄haloalkynyloxy-C₁-C₂alkyl, C₃-C₅alkylthio, C₃-C₅alkylthio-C₁-C₂alkyl, C₂-C₄alkylthio-C₂-C₃alkoxy or C₂-C₄alkylthio-C₂-C₃alkylthio;
R₂ is chlorine or bromine;
R₃ is H, halogen or methyl;
R₄ is H or methyl;
R₅ is C₁-C₈alkyl, C₃-C₆cycloalkyl, C₂-C₄alkenyl, C₂-C₃alkynyl, methoxyethyl, cyanomethyl, haloethyl, halovinyl, haloacetylenyl, trifluoromethylphenyl, benzyl or substituted benzyl, the substituents being selected from the group consisting of methyl, methoxy and chlorine; and
R₆ is H, pyridyl, halopyridyl, furyl, or R₅ and R₆ together are C₃-C₅alkylene.

6. A compound according to claim 1, of the formula I in which
R₁ is C₄-C₆alkyl, cyclohexyl, C₄-C₅alkynyl, C₄-C₅alkoxy, C₅-C₆cycloalkoxy, C₂-C₄alkoxy-C₁-C₄alkyl, C₁-C₂alkoxyethoxy, C₅-C₆cycloalkoxymethyl, C₄-C₆alkenyloxy, C₃-C₅alkenyloxymethyl, C₃-C₅alkynyloxy, C₃-C₄haloalkoxy, C₃haloalkenyloxy, C₃haloalkenyloxymethyl, C₃haloalkynyloxy, C₃haloalkynyloxymethyl, C₄alkylthio, C₄alkylthiomethyl, C₂-C₃alkylthioethoxy or C₂-C₃alkylthioethylthio;
R₂ is chlorine or bromine;
R₃ is H, halogen or methyl;
R₄ is H or methyl;
R₅ is C₁-C₈alkyl, C₃-C₆cycloalkyl, C₂-C₄alkenyl, C₂-C₃alkynyl, methoxyethyl, cyanomethyl, haloethyl, halovinyl, haloacetylenyl, trifluoromethylphenyl, benzyl or substituted benzyl, the substituents being selected from the group consisting of methyl, methoxy and chlorine; and
R₆ is H, pyridyl, halopyridyl, furyl, or R₅ and R₆ together are C₃-C₅alkylene.

7. A compound according to claim 1, of the formula I in which
R₁ is C₄-C₆alkyl, cyclohexyl, C₄-C₅alkynyl, C₄-C₅alkoxy, C₅-C₆cycloalkoxy, C₂-C₄alkoxy-C₁-C₄alkyl, C₁-C₂alkoxyethoxy, C₅-C₆cycloalkoxymethyl, C₄-C₆alkenyloxy, C₃-C₅alkenyloxymethyl, C₃-C₅alkynyloxy, C₃-C₄haloalkoxy, C₃haloalkenyloxy, C₃haloalkenyloxymethyl, C₃haloalkynyloxy, C₃haloalkynyloxymethyl, C₄alkylthio, C₄alkylthiomethyl, C₂-C₃alkylthioethoxy or C₂-C₃alkylthioethylthio;
R₂ is chlorine or bromine;
R₃ is H, halogen or methyl;
R₄ is H or methyl;
R₅ is C₁-C₈alkyl, cyclopropyl, C₂-C₄alkenyl, C₂-C₃alkynyl, methoxyethyl, cyanomethyl, fluoroethyl, chloroethyl, fluorovinyl, chlorovinyl, bromovinyl, iodacetylenyl, trifluoromethylphenyl, benzyl, tolyl, anisyl or chlorophenyl; and
R₆ is H, pyridyl, halopyridyl, furyl, or R₅ and R₆ together are C₃-C₅alkylene.

8. A compound according to claim 1, of the formula I in which
R₁ is C₄-C₆alkyl, cyclohexyl, C₄-C₅alkynyl, C₄-C₅alkoxy, C₅-C₆cycloalkoxy, C₂-C₄alkoxy-C₁-C₄alkyl, C₁-C₂alkoxyethoxy, C₅-C₆cycloalkoxymethyl, C₄-C₆alkenyloxy, C₃-C₅alkenyloxymethyl, C₃-C₅alkynyloxy, C₃-C₄haloalkoxy, C₃haloalkenyloxy, C₃haloalkenyloxymethyl, C₃haloalkynyloxy, C₃haloalkynyloxymethyl, C₄alkylthio, C₄alkylthiomethyl, C₂-C₃alkylthioethoxy or C₂-C₃alkylthioethylthio;
R₂ is chlorine or bromine;
R₃ is H, halogen or methyl;
R₄ is H or methyl;
R₅ is C₁-C₈alkyl, cyclopropyl, C₂-C₄alkenyl, C₂-C₃alkynyl, methoxyethyl, cyanomethyl, fluoroethyl, chloroethyl, fluorovinyl, chlorovinyl, bromovinyl, iodacetylenyl, trifluoromethylphenyl, benzyl, tolyl, anisyl or chlorophenyl; and
R₆ is H, pyridyl, chloropyridyl, furyl, or R₅ and R₆ together are C₃-C₅alkylene.

9. A compound according to claim 1, of the formula I in which
R₁ is sec-butoxy or isobutoxy;
R₂ is chlorine or bromine;
R₃ is H or bromine;
R₄ and R₆ are H; and
R₅ is ethyl or propyl.

10. A process for the preparation of a compound according to claim 1, of the formula I, which comprises
a) reacting a compound of the formula 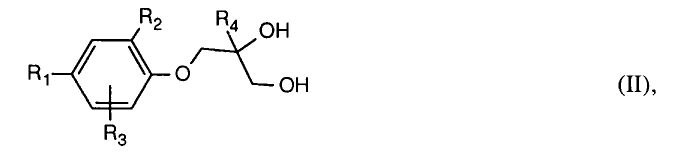 which is known or can be prepared in analogy to corresponding known compounds and in which R₁, R₂, R₃ and R₄ are as defined for formula I, in free form or in salt form, with a compound of the formula
R₅COR₆ (III),
which is known or can be prepared in analogy to corresponding known compounds and in which R₅ and R₆ are as defined for formula I, or with a compound of the formula 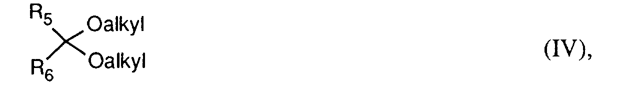 which is known or can be prepared in analogy to corresponding known compounds and in which R₅ and R₆ are as defined for formula I and alkyl is methyl or ethyl, if appropriate in the presence of an acid catalyst or a dehydrating agent, or
b) reacting a compound of the formula 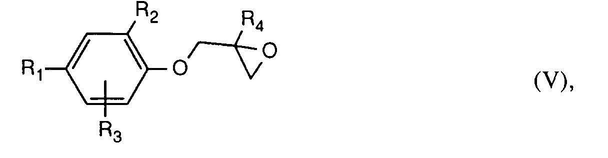 which is known or can be prepared in analogy to corresponding known compounds and in which R₁, R₂, R₃ and R₄ are as defined for formula I, with a compound of the formula III, if appropriate in the presence of an acid catalyst or a dehydrating agent, or
c) reacting a compound of the formula 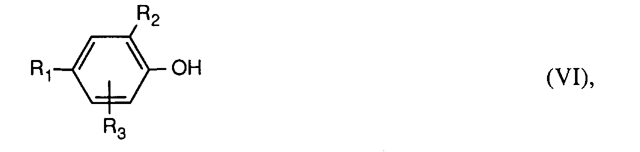 which is known or can be prepared in analogy to corresponding known compounds and in which R₁, R₂ and R₃ are as defined for formula I, with a compound of the formula 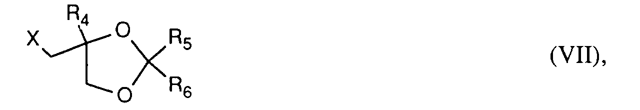 which is known or can be prepared in analogy to corresponding known compounds and in which R₄, R₅ and R₆ are as defined for formula I and X is a leaving group, if appropriate in the presence of a base or a dehydrating agent,
and/or, if desired, converting a compound of the formula I which can be obtained in accordance with the process or by a different route into a different compound of the formula I, and/or separating an isomer mixture which can be obtained in accordance with the process and isolating the desired isomer.

11. A pesticide which comprises at least one compound according to claim 1, of the formula I, as active ingredient and, if desired, at least one auxiliary.

12. A process for the preparation of a composition according to claim 11 and comprising at least one auxiliary, which comprises intimately mixing and/or grinding the active ingredient with the auxiliary(ies).

13. The use of a compound according to claim 1, of the formula I, for the preparation of a composition according to claim 11.

14. The use of a composition according to claim 11 for controlling pests.

15. The use according to claim 14 for protecting plant propagation material.

16. A method of controlling pests, which comprises applying a composition according to claim 11 to the pests or their environment.

17. A method according to claim 16 for protecting plant propagation material, which comprises treating the propagation material or the locus where the propagation material is planted.

18. Plant propagation material treated in accordance with the method described in claim 17.

## Revendications

1. Composé de formule 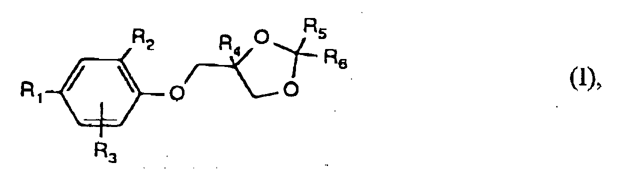 dans laquelle
R₁ représente un alkyle en C₁ à C₈ éventuellement substitué, un cycloalkyle en C₃ à C₈ éventuellement substitué, un alcényle en C₂ à C₈ éventuellement substitué, un alcynyle en C₂ à C₈ éventuellement substitué, un alcoxy en C₁ à C₈ éventuellement substitué, un cycloalcoxy en C₃ à C₈ éventuellement substitué, un alcényloxy en C₂ à C₈ éventuellement substitué, un alcynyloxy en C₂ à C₈ éventuellement substitué ou un alkylthio en C₁ à C₈ eventuellement substitué ;
R₂ représente le chlore ou le brome ;
R₃ représente H, halogène ou méthyle ;
R₄ représente H ou méthyle ;
R₅ représente un alkyle en C₁ à C₈, cycloalkyle en C₃ à C₆, alcényle en C₂ à C₅, alcynyle en C₂ à C₅, alcoxy en C₁ à C₃-alkyle en C₁ à C₃, cyanoalkyle en C₁ à C₄, halogénoalkyle en C₁ à C₆, halogénoalcényle en C₂ à C₅, halogénoalcynyle en C₂ à C₃, alcoxycarbonyle en C₁ à C₃, trifluorométhylphényle, benzyle ou benzyle substitué, où les substituants sont choisis dans le groupe constitué par alkyle en C₁ à C₃, alcoxy en C₁ à C₃ et halogène ; et
R₆ représente H, un pyridyle, halogénopyridyle, furyle, thiényle, ou R₅ et R₆ représentent ensemble un alkylène en C₃ à C₅ à chaîne droite.

2. Composé selon la revendication 1 de formule I, dans laquelle
R₁ représente un alkyle en C₁ à C₈, cycloalkyle en C₃ à C₈, alcényle en C₂ à C₈, alcynyle en C₂ à C₈, alcoxy en C₁ à C₈, cycloalcoxy en C₃ à C₈, alcoxy en C₂ à C₈-alkyle en C₁ à C₄, alcoxy en C₁ à C₈-alcoxy en C₂ à C₄-alkyle en C₁ à C₄, alcoxy en C₁ à C₈-alcoxy en C₂ à C₄, cycloalcoxy en C₄ à C₈-alkyle en C₁ à C₃, alcényloxy en C₃ à C₈, alcényloxy en C₃ à C₈-alkyle en C₁ à C₃, alcynyloxy en C₃ à C₈, alcynyloxy en C₃ à C₈-alkyle en C₁ à C₃, halogénoalkyle en C₃ à C₈, halogénoalcényle en C₃ à C₈, halogénoalcynyle en C₃ à C₈, halogénoalcoxy en C₃ à C₈, halogénoalcoxy en C₃ à C₈-alkyle en C₁ à C₃, halogénoalcényloxy en C₃ à C₈, halogénoalcényloxy en C₃ à C₈-alkyle en C₁ à C₃, halogène en C₃ à C₈-alcynyloxy, halogénalcynyloxy en C₃ à C₈-alkyle en C₁ à C₃, alkylthio en C₃ à C₈, alkylthio en C₃ à C₈-alkyle en C₁ à C₃, alkylthio en C₂ à C₈-alcoxy en C₂ à C₃, alkylthio en C₂ à C₈-alcoxy en C₂ à C₃-alkyle en C₁ à C₄, alkylthio en C₂ à C₈-alkylthio en C₂ à C₃, alkylthio en C₂ à C₈-alkylthio en C₂ à C₃-alkyle en C₂ à C₄, alcoxy en C₂ à C₈-alkylthio en C₂ à C₃ ou alcoxy en C₂ à C₈-alkylthio en C₂ à C₃-alkyle en C₁ à C₄ ;
R₂ représente le chlore ou le brome ;
R₃ représente H, halogène ou méthyle ;
R₄ représente H ou méthyle ;
R₅ représente un alkyle en C₁ à C₈, cycloalkyle en C₃ à C₆, alcényle en C₂ à C₄, alcynyle en C₂ à C₃, alcoxy en C₁ à C₃-alkyle en C₁ à C₃, cyanoalkyle en C₂ à C₄, halogénoalkyle en C₂ à C₆, halogénalcényle en C₂ à C₅, halogénoalcynyle en C₂ à C₃, alcoxycarbonyle en C₁ à C₃, trifluorométhylphényle, benzyle ou benzyle substitué, où les substituants sont choisis dans le groupe constitué par alkyle en C₁ à C₃, alcoxy en C₁ à C₃ et chlore et ;
R₆ représente H, un pyridyle, halogénopyridyle, furyle ou R₅ et R₆ représentent ensemble un alkylène en C₃ à C₅.

3. Composé selon la revendication 1 de formule I, dans laquelle
R₁ représente un alkyle en C₃ à C₈, cycloalkyle en C₃ à C₈, alcényle en C₃ à C₈, alcynyle en C₃ à C₈, alcoxy en C₃ à C₈, cycloalcoxy en C₃ à C₈, alcoxy en C₂ à C₈-alkyle en C₁ à C₄, alcoxy en C₁ à C₈-alcoxy en C₂ à C₄-alkyle en C₁ à C₄, alcoxy en C₁ à C₈-alcoxy en C₂ à C₄, cycloalcoxy en C₄ à C₈-alkyle en C₁ à C₃, alcényloxy en C₃ à C₈, alcényloxy en C₃ à C₈-alkyle en C₁ à C₃, alcynyloxy en C₃ à C₈, alcynyloxy en C₃ à C₈-alkyle en C₁ à C₃, halogénoalkyle en C₃ à C₈, halogénoalcényle en C₃ à C₈, halogénoalcynyle en C₃ à C₈, halogénoalcoxy en C₃ à C₈, halogénoalcoxy en C₃ à C₈-alkyle en C₁ à C₃, halogénoalcényloxy en C₃ à C₈, halogénoalcényloxy en C₃ à C₈-alkyle en C₁ à C₃, halogène en C₃ à C₈-alcynyloxy, halogénoalcynyloxy en C₃ à C₈-alkyle en C₁ à C₃, alkylthio en C₃ à C₈, alkylthio en C₃ à C₈-alkyle en C₁ à C₃, alkylthio en C₂ à C₈-alcoxy en C₂ à C₃, alkylthio en C₂ à C₈-alcoxy en C₂ à C₃-alkyle en C₁ à C₄, alkylthio en C₂ à C₈-alkylthio en C₂ à C₃, alkylthio en C₂ à C₈-alkylthio en C₂ à C₃-alkyle en C₁ à C₄, alcoxy en C₂ à C₈-alkylthio en C₂ à C₃ ou alcoxy en C₂ à C₈-alkylthio en C₂ à C₃-alkyle en C₁ à C₄ ;
R₂ représente le chlore ou le brome ;
R₃ représente H, halogène ou méthyle ;
R₄ représente H ou méthyle ;
R₅ représente un alkyle en C₁ à C₈, cycloalkyle en C₃ à C₆, alcényle en C₂ à C₅, alcynyle en C₂ à C₅, alcoxy en C₁ à C₃-alkyle en C₁ à C₃, cyanoalkyle en C₂ à C₄, halogénoalkyle en C₂ à C₆, halogénoalcényle en C₂ à C₅, halogénoalcynyle en C₂ à C₃, alcoxycarbonyle en C₁ à C₃, trifluorométhylphényle, benzyle ou benzyle substitué, où les substituants sont choisis dans le groupe constitué par alkyle en C₁ à C₃, alcoxy en C₁ à C₃ et halogène et ;
R₆ représente H, un pyridyle, halogénopyridyle, furyle, ou R₅ et R₆ représentent ensemble un alkylène en C₃ à C₅ à chaîne droite.

4. Composé selon la revendication 1 de formule I, dans laquelle
R₁ représente un alkyle en C₃ à C₆, cycloalkyle en C₃ à C₆, alcényle en C₃ à C₆, alcynyle en C₃ à C₆, alcoxy en C₃ à C₈, cycloalcoxy en C₃ à C₆, alcoxy en C₂ à C₆-alkyle en C₁ à C₄, alcoxy en C₁ à C₆-alcoxy en C₂ à C₄, cycloalcoxy en C₄ à C₆-alkyle en C₁ à C₃, alcényloxy en C₃ à C₆, alcényloxy en C₃ à C₆-alkyle en C₁ à C₃, alcynyloxy en C₃ à C₆, alcynyloxy en C₃ à C₆-alkyle en C₁ à C₃, halogénoalkyle en C₃ à C₆, halogénoalcényle en C₃ à C₆, halogénoalcynyle en C₃ à C₆, halogénoalcoxy en C₃ à C₆, halogénoalcoxy en C₃ à C₆-alkyle en C₁ à C₃, halogénoalcényloxy en C₃ à C₆, halogénoalcényloxy en C₃ à C₆-alkyle en C₁ à C₃, halogénoalcynyloxy en C₃ à C₆, halogénoalcynyloxy en C₃ à C₆-alkyle en C₁ à C₃, alkylthio en C₃ à C₆, alkylthio en C₃ à C₆-alkyle en C₁ à C₃, alkylthio en C₂ à C₆-alcoxy en C₂ à C₃ ou alkylthio en C₂ à C₆-alkylthio en C₂ à C₃ ;
R₂ représente le chlore ou le brome ;
R₃ représente H, halogène ou méthyle ;
R₄ représente H ou méthyle ;
R₅ représente un alkyle en C₁ à C₈, cycloalkyle en C₃ à C₆, alcényle en C₂ à C₄, alcynyle en C₂ à C₃, alcoxy en C₁ à C₃-alkyle en C₁ à C₃, cyanoalkyle en C₂ à C₄, halogénoalkyle en C₂ à C₆, halogénoalcényle en C₂ à C₅, halogénoalcynyle en C₂ à C₃, alcoxycarbonyle en C₁ à C₃, trifluorométhylphényle, benzyle ou benzyle substitué, où les substituants sont choisis dans le groupe constitué par alkyle en C₁ à C₃, alcoxy en C₁ à C₃ et chlore et ;
R₆ représente H, un pyridyle, halogénopyridyle, furyle, ou R₅ et R₆ représentent ensemble un alkylène en C₃ à C₅.

5. Composé selon la revendication 1 de formule I, dans laquelle
R₁ représente un alkyle en C₄ à C₆, cycloalkyle en C₄ à C₆, alcényle en C₃ à C₆, alcynyle en C₂ à C₅, alcoxy en C₄ à C₈, cycloalcoxy en C₅ à C₆, alcoxy en C₂ à C₄-alkyle en C₁ à C₄, alcoxy en C₁ à C₃-alcoxy en C₂ à C₄, cycloalcoxy en C₅ à C₆-alkyle en C₁ à C₂, alcényloxy en C₄ à C₆, alcényloxy en C₃ à C₅-alkyle en C₁ à C₂, alcynyloxy en C₃ à C₅, alcynyloxy en C₃ à C₅-alkyle en C₁ à C₂, halogénoalkyle en C₃ à C₅, halogénoalcényle en C₃ à C₅, halogénoalcynyle en C₃ à C₅, halogénoalcoxy en C₃ à C₅, halogénoalcoxy en C₃ à C₅-alkyle en C₁ à C₂, halogénoalcényloxy en C₃ à C₄, halogénoalcényloxy en C₃ à C₄-alkyle en C₁ à C₂, halogénoalcynyloxy en C₃ à C₄, halogénoalcynyloxy en C₃ à C₄-alkyle en C₁ à C₂, alkylthio en C₃ à C₅, alkylthio en C₃ à C₅-alkyle en C₁ à C₂, alkylthio en C₂ à C₄-alcoxy en C₂ à C₃, ou alkylthio en C₂ à C₄-alkylthio en C₂ à C₃ ;
R₂ représente le chlore ou le brome ;
R₃ représente H, halogène ou méthyle ;
R₄ représente H ou méthyle ;
R₅ représente un alkyle en C₁ à C₈, cycloalkyle en C₃ à C₆, alcényle en C₂ à C₄, alcynyle en C₂ à C₃, méthoxyéthyle, cyanométhyle, halogénoéthyle, halogénovinyle, halogénoacétylényle, trifluorométhylphényle, benzyle ou benzyle substitué, où les substituants sont choisis dans le groupe constitué par méthyle, méthoxy et chlore ; et
R₆ représente H, un pyridyle, halogénopyridyle, furyle ou R₅ et R₆ représentent ensemble un alkylène en C₃ à C₅.

6. Composé selon la revendication 1, de formule I, dans laquelle
R₁ représente un alkyle en C₄ à C₆, cyclohexyle, alcynyle en C₄ à C₅, alcoxy en C₄ à C₅, cycloalcoxy en C₅ à C₆, alcoxy en C₂ à C₄-alkyle en C₁ à C₄, alcoxyéthoxy en C₁ à C₂, cycloalcoxyméthyle en C₅ à C₆, alcényloxy en C₄ à C₆, alcényloxyméthyle en C₃ à C₅, alcynyloxy en C₃ à C₅, halogène en C₃ à C₄-alcoxy, halogénoalcényloxy en C₃, halogénoalcényloxyméthyle en C₃, halogénoalcynyloxy en C₃, halogénoalcynyloxyméthyle en C₃, alkylthio en C₄, alkylthiométhyle en C₄, alkylthioéthoxy en C₂ à C₃ ou alkylthioéthylthio en en C₂ à C₃ ;
R₂ représente le chlore ou le brome ;
R₃ représente H, halogène ou méthyle ;
R₄ représente H ou méthyle ;
R₅ représente un alkyle en C₁ à C₈, cycloalkyle en C₃ à C₆, alcényle en C₂ à C₄, alcynyle en C₂ à C₃, méthoxyéthyle, cyanométhyle, halogénoéthyle, halogénovinyle, halogénoacétylényle, trifluorométhylphényle, benzyle ou benzyle substitué, où les substituants sont choisis dans le groupe constitué par méthyle, méthoxy et chlore ; et
R₆ représente H, un pyridyle, halogénopyridyle, furyle ou R₅ et R₆ représentent ensemble un alkylène en C₃ à C₅.

7. Composé selon la revendication 1, de formule I, dans laquelle
R₁ représente un alkyle en C₄ à C₆, cyclohexyle, alcynyle en C₄ à C₅, alcoxy en C₄ à C₅, cycloalcoxy en C₅ à C₆, alcoxy en C₂ à C₄-alkyle en C₁ à C₄, alcoxyéthoxy en C₁ à C₂, cycloalcoxyméthyle en C₅ à C₆, alcényloxy en C₄ à C₆, alcényloxyméthyle en C₃ à C₅, alcynyloxy en C₃ à C₅, halogénoalcoxy en C₃ à C₄, halogénoalcényloxy en C₃, halogénoalcényloxyméthyle en C₃, halogénoalcynyloxy en C₃, halogénoalcynyloxyméthyle en C₃, alkylthio en C₄, alkylthiométhyle en C₄, alkylthioéthoxy en C₂ à C₃, ou alkylthioéthylthio en C₂ à C₃ ;
R₂ représente le chlore ou le brome ;
R₃ représente H, halogène ou méthyle ;
R₄ représente H ou méthyle ;
R₅ représente un alkyle en C₁ à C₈, cyclopropyle, alcényle en C₂ à C₄, alcynyle en C₂ à C₃, méthoxyéthyle, cyanométhyle, fluoroéthyle, chloroéthyle, fluorovinyle, chlorovinyle, bromovinyle, iodoacétylényle, trifluorométhylphényle, benzyle, tolyle, anisyle ou chlorophényle ; et
R₆ représente H, un pyridyle, halogénopyridyle, furyle, ou R₅ et R₆ représentent ensemble un alkylène en C₃ à C₅.

8. Composé selon la revendication 1, de formule I, dans laquelle
R₁ représente un alkyle en C₄ à C₆, cyclohexyle, alcynyle en C₄ à C₅, alcoxy en C₄ à C₅, cycloalcoxy en C₅ à C₆, alcoxy en C₂ à C₄-alkyle en C₁ à C₄, alcoxyéthoxy en C₁ à C₂, cycloalcoxyméthyle en C₅ à C₆, alcényloxy en C₄ à C₆, alcényloxyméthyle en C₃ à C₅, alcynyloxy en C₃ à C₅, halogénoalcoxy en C₃ à C₄, halogénoalcényloxy en C₃, halogénoalcényloxyméthyle en C₃, halogénoalcynyloxy en C₃, halogénoalcynyloxyméthyle en C₃, alkylthio en C₄, alkylthiométhyle en C₄, alkylthioéthoxy en C₂ à C₃ ou alkylthioéthylthio en C₂ à C₃ ;
R₂ représente le chlore ou le brome ;
R₃ représente H, halogène ou méthyle ;
R₄ représente H ou méthyle ;
R₅ représente un alkyle en C₁ à C₈, cyclopropyle, alcényle en C₂ à C₄, alcynyle en C₂ à C₃, méthoxyéthyle, cyanométhyle, fluoroéthyle, chloroéthyle, fluorovinyle, chlorovinyle, bromovinyle, iodoacétylényle, trifluorométhylphényle, benzyle, tolyle, anisyle ou chlorophényle ; et
R₆ représente H, un pyridyle, chloropyridyle, furyle, ou R₅ et R₆ représentent ensemble un alkylène en C₃ à C₅.

9. Composé selon la revendication 1 de formule I, dans laquelle
R₁ représente un sec.-butoxy ou iso-butoxy ;
R₂ représente le chlore ou le brome ;
R₃ représente H ou le brome ;
R₄ et R₆ représentent H ; et
R₅ représente un éthyle ou propyle.

10. Procédé de préparation d'un composé selon la revendication 1 de formule I, caractérisé en ce que
a) on fait réagir un composé de formule 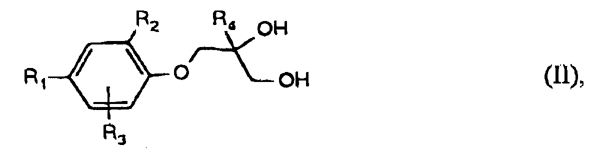 qui est connu ou que l'on peut préparer par analogie avec des composés connus correspondants et où R₁, R₂, R₃ et R₄ ont les significations indiquées pour la formule I, sous forme libre ou sous forme de sel, éventuellement en présence d'un catalyseur acide ou d'un agent fixant l'eau, avec un composé de formule
R₅COR₆ (III),
qui est connu ou que l'on peut préparer par analogie avec des composés connus correspondants, et où R₅ et R₆ ont les significations données pour la formule I, ou avec un composé de formule 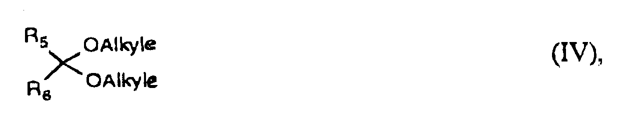 qui est connu ou que l'on peut préparer par analogic avec des composés connus correspondants, et où R₅ et R₆ ont les significations indiquées pour la formule I et alkyle est un méthyle ou un éthyle, ou
b) on fait réagir un composé de formule 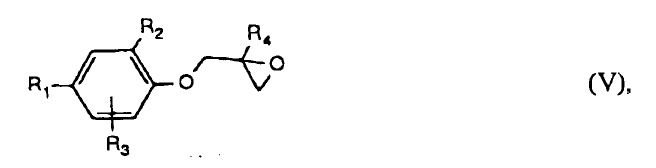 qui est connu ou que l'on peut préparer par analogie avec des composés connus correspondants et où R₁, R₂, R₃ et R₄ ont les significations indiquées pour la formule I, éventuellement en présence d'un catalyseur acide ou d'un agent fixant l'eau, avec un composé de formule III, ou
c) on fait réagir un composé de formule 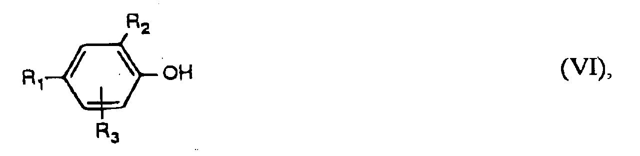 qui est connu ou que l'on peut préparer par analogie avec des composés connus correspondants et où R₁, R₂ et R₃ ont les significations indiquées pour la formule I, éventuellement en présence d'une base ou d'un agent fixant l'eau, avec un composé de formule 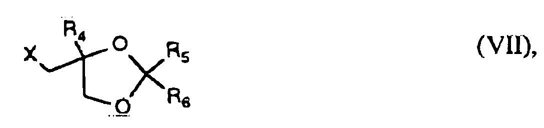 qui est connu ou que l'on peut préparer par analogie avec des composés connus correspondants et où R₄, R₅ et R₆ ont les significations données pour la formule 1 et X est un groupe sortant,
et/ou, si on le désire, en ce qu'on transforme un composé de formule I que l'on peut obtenir selon le procédé ou d'une autre manière, en un autre composé de formule I et/ou en ce qu'on sépare un mélange d'isomères que l'on peut obtenir selon le procédé et en ce qu'on isole l'isomère désiré.

11. Agent de lutte antiparasitaire, caractérisé en ce qu'il contient au moins un composé selon la revendication 1 comme matière active et le cas échéant au moins un additif.

12. Procédé de préparation d'un agent selon la revendication 11 contenant au moins un additif, caractérisé en ce qu'on mélange intimement et/ou en ce qu'on broie la matière active avec le ou les additifs.

13. Utilisation d'un composé selon la revendication 1 de formule I pour préparer un agent selon la revendication 11.

14. Utilisation d'un agent selon la revendication 11 pour la lutte contre les parasites.

15. Utilisation selon la revendication 14 pour la protection des parties reproductrices des plantes.

16. Procédé de lutte contre les parasites, caractérisé en ce qu'on applique sur les parasites ou leurs lieux de vie un agent selon la revendication 11.

17. Procédé selon la revendication 16 de protection de la partie reproductrice des plantes, caractérisé en ce qu'on traite la partie reproductrice ou l'endroit de culture de la partie reproductrice.

18. Partie reproductrice de plante traitée selon le procédé décrit dans la revendication 17.
